# EUROPEAN PATENT APPLICATION

(11) **EP 4 265 249 A1**
(43) Date of publication of application: **25.10.2023**
(21) Application number: 21888664.6
(22) Date of filing: 05.11.2021
(51) Int. Cl.: A61K 31/4375, C07D 455/03, A61P 35/00, C12Q 1/6886

(54) **APPLICATION OF ISOQUINOLINE COMPOUND IN TUMOR TREATMENT**

(30) Priority: 06.11.2020 CN 202011233335
(71) Applicant: Nanjing Shijiang Medicine Technology Co., Ltd, Nanjing, Jiangsu 200120 (CN); Tongji University, Shanghai 200092 (CN)
(72) Inventor: SHI, Yufeng, Nanjing, Jiangsu 200120 (CN); MA, Wenjiang, Nanjing, Jiangsu 200120 (CN); JIANG, Cizhong, Shanghai 200092 (CN); WU, Changqing, Shanghai 200092 (CN); LIU, Yu'e, Shanghai 200092 (CN); LIU, Wenju, Shanghai 200092 (CN)
(74) Representative: reuteler & cie SA
(86) International application number: PCT/CN2021/129066
(87) International publication number: WO 2022/095972

(57) **Abstract**

The present invention relates to an application of an isoquinoline compound in tumor treatment. Specifically, the present invention provides a use of a compound of formula I, or an optical isomer or a racemate thereof, or a solvate thereof, or a pharmaceutically acceptable salt thereof used for the preparation of a composition or formulation for the prevention and/or treatment of a tumor. The compound of the present invention has significant and exceptional therapeutic effects on tumors with low or no expression of an NNMT gene, high expression of a DNA methylase, high expression of UHRF1, high methylation levels at an NNMT gene nucleotide site, and/or high methylation levels at a DNA CpG site in an NNMT gene region.

## Description

### FIELD OF THE INVENTION

The present invention relates to the field of medicine. Specifically, the present invention relates to application of isoquinoline compound in tumor treatment

### BACKGROUND TECHNOLOGY

Tumor is a common disease that seriously endangers human health, and the mortality rate of malignant tumors is rising. Due to the heterogeneity of tumor and individual difference in patient, simply using the same treatment method or medication based on source or pathological characteristic of tumors can easily lead to improper treatment, which can delay valuable treatment time and opportunities for patient. Therefore, it is necessary to take personalized treatment according to the different situations of patient. With the development of biological technology, the treatment of tumor has entered the era of precision treatment, and more and more changes in tumor-related gene expression have been discovered. Changes in related genes play an important role in the development of malignant tumors, such as up-regulating the specific function of cell to promote cancer cell immortality. The discovery and application of biomarkers can provide precise guidance for the application of related drug and make personalized treatment of tumor possible, thereby achieving targeted administration of drug and significantly improving treatment effect.

Therefore, there is an urgent need in the art to develop a drug that can achieve precise treatment of tumor.

### SUMMARY OF THE INVENTION

It is an object of the present invention to provide a marker for determining whether the compound of present invention is suitable for use in the prevention and/or treatment of patient tumor to achieve precise treatment of tumor, the marker is the expression level of NNMT gene, the expression level of DNA methylase, the expression level of UHRF1, the methylation level of nucleotide site of NNMT gene, and/or the methylation level of DNA CpG site of NNMT gene. The compound of present invention has more significant treatment effect on tumors with low or no expression of NNMT gene, high expression of DNA methylase, high expression of UHRF1, high methylation level of nucleotide site of NNMT gene, and/or high methylation level of DNA CpG site of NNMT gene.

In the first aspect of the present invention, it provides a use of a compound of formula I, or an optical isomer thereof, or a racemate thereof, or a solvate thereof, or a pharmaceutically acceptable salt thereof in the preparation of a composition or a preparation for preventing and/or treating tumor; 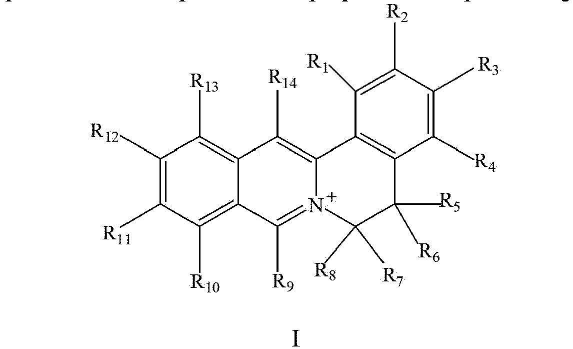 wherein,
R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C12 alkyl, substituted or unsubstituted C3-C12 cycloalkyl, substituted or unsubstituted C1-C12 alkoxyl, substituted or unsubstituted C1-C12 alkylthio, substituted or unsubstituted 3-12 membered heterocycloalkyl, substituted or unsubstituted C6-C12 aryl, substituted or unsubstituted 3-12 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring;
R₅, R₆, R₇, R₈, R₉ and R₁₄ are each independently hydrogen, halogen, - CN, hydroxyl, sulfhydryl, nitro, amino, -COOH, -CHO, substituted or unsubstituted C1-C12 alkyl, substituted or unsubstituted C3-C12 cycloalkyl, substituted or unsubstituted C1-C12 alkoxyl, substituted or unsubstituted C1-C12 alkylthio, substituted or unsubstituted 3-12 membered heterocycloalkyl, substituted or unsubstituted C6-C12 aryl, or substituted or unsubstituted 3-12 membered heteroaryl;
each "substituted" means that one or more (preferably 1, 2, 3, or 4) hydrogen atoms on the group are substituted by a substituent selected from the group consisting of C1-C8 alkyl, C3-C8 cycloalkyl, C2-C4 alkenyl, C2-C4 alkynyl, C1-C8 haloalkyl, C3-C8 halocycloalkyl, halogen, nitro, -CN, carbonyl (=O), cyano, hydroxyl, sulfhydryl, amino, C1-C4 carboxyl, C2-C8 ester group, C2-C4 amide group, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, 5-10 membered heterocycloalkyl, pyrrolidine-2,5-diketone group;
the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

In another preferred embodiment, the cycloalkane ring contains 1, 2, or 3 C=C ring double bond.

In another preferred embodiment, the heterocycloalkane ring contains 1, 2, or 3 C=C ring double bonds.

In another preferred embodiment, each "substituted" means that one or more (preferably 1, 2, 3, or 4) hydrogen atoms on the group are substituted by a substituent selected from the group consisting of C1-C6 alkyl, C3-C8 cycloalkyl, C2-C4 alkenyl, C2-C4 alkynyl, C1-C6 haloalkyl, C3-C8 halocycloalkyl, halogen, nitro, -CN, carbonyl (=O), cyano, hydroxyl, sulfhydryl, amino, C1-C4 carboxyl, C2-C6 ester group, C2-C4 amide group, C1-C6 alkoxyl, C1-C6 alkylthio, C3-C6 cycloalkoxyl, C3-C6 cycloalkylthio, C1-C6 haloalkoxyl, C1-C6 haloalkylthio, C6-C12 aryl, 5-10 membered heteroaryl, 5-10 membered heterocycloalkyl, pyrrolidine-2,5-diketone group.

In another preferred embodiment, each "substituted" means that one or more (preferably 1, 2, 3, or 4) hydrogen atoms on the group are substituted by a substituent selected from the group consisting of C1-C4 alkyl, C3-C8 cycloalkyl, C2-C4 alkenyl, C2-C4 alkynyl, C1-C4 haloalkyl, C3-C8 halocycloalkyl, halogen, nitro, -CN, carbonyl (=O), cyano, hydroxyl, sulfhydryl, amino, C1-C4 carboxyl, C2-C6 ester group, C2-C4 amide group, C1-C4 alkoxyl, C1-C4 alkylthio, C3-C6 cycloalkoxyl, C3-C6 cycloalkylthio, C1-C4 haloalkoxyl, C1-C4 haloalkylthio, C6-C12 aryl, 5-10 membered heteroaryl, 5-10 membered heterocycloalkyl, pyrrolidine-2,5-diketone group.

In another preferred embodiment, each "substituted" means that one or more (preferably 1, 2, 3, or 4) hydrogen atoms on the group are substituted by a substituent selected from the group consisting of C1-C4 alkyl, C2-C4 alkenyl, C2-C4 alkynyl, halogen, C1-C4 alkoxyl, C1-C4 carboxyl, C2-C6 amide group.

In another preferred embodiment, the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

In another preferred embodiment, R₁ and R₂, R₂ and R₃, R₃ and R₄, R₁₀ and R₁₁, R₁₁ and R₁₂, and R₁₂ and R₁₃ each independently form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₁ and R₂ form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₂ and R₃ form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₃ and R₄ form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₁₀ and Rn form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₁₁ and R₁₂ form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₁₂ and R₁₃ form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring.

In another preferred embodiment, R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C8 alkyl, substituted or unsubstituted C3-C10 cycloalkyl, substituted or unsubstituted C1-C10 alkoxyl, substituted or unsubstituted C1-C10 alkylthio, substituted or unsubstituted 3-10 membered heterocycloalkyl, substituted or unsubstituted C6-C12 aryl, substituted or unsubstituted 3-10 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C8 cycloalkane ring, substituted or unsubstituted 3-10 membered heterocycloalkane ring, substituted or unsubstituted C6-C12 aromatic ring, or substituted or unsubstituted 3-10 membered heteroaromatic ring.

In another preferred embodiment, R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C8 alkyl, substituted or unsubstituted C3-C10 cycloalkyl, substituted or unsubstituted C1-C8 alkoxyl, substituted or unsubstituted C1-C8 alkylthio, substituted or unsubstituted 3-10 membered heterocycloalkyl, substituted or unsubstituted C6-C12 aryl, substituted or unsubstituted 3-10 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C8 cycloalkane ring, substituted or unsubstituted 3-10 membered heterocycloalkane ring, substituted or unsubstituted C6-C12 aromatic ring, or substituted or unsubstituted 3-10 membered heteroaromatic ring.

In another preferred embodiment, R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C6 alkyl, substituted or unsubstituted C3-C10 cycloalkyl, substituted or unsubstituted C1-C6 alkoxyl, substituted or unsubstituted C1-C6 alkylthio, substituted or unsubstituted 3-10 membered heterocycloalkyl, substituted or unsubstituted C6-C12 aryl, substituted or unsubstituted 3-10 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C8 cycloalkane ring, substituted or unsubstituted 3-10 membered heterocycloalkane ring, substituted or unsubstituted C6-C12 aromatic ring, or substituted or unsubstituted 3-10 membered heteroaromatic ring.

In another preferred embodiment, R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, substituted or unsubstituted 3-8 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C6 cycloalkane ring, substituted or unsubstituted 3-8 membered heterocycloalkane ring, substituted or unsubstituted C6-C12 aromatic ring, or substituted or unsubstituted 3-8 membered heteroaromatic ring.

In another preferred embodiment, R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, substituted or unsubstituted 3-8 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C6 cycloalkane ring, substituted or unsubstituted 5-7 membered heterocycloalkane ring, substituted or unsubstituted C6-C12 aromatic ring, or substituted or unsubstituted 3-8 membered heteroaromatic ring.

In another preferred embodiment, R₁, R₂, R₃, R₄, R₁₀, R₁₁, R₁₂ and R₁₃ are each independently hydrogen, substituted or unsubstituted C1-C6 alkyl, substituted or unsubstituted C1-C6 alkoxyl, substituted or unsubstituted C1-C6 alkylthio; or R₁ and R₂, R₂ and R₃, R₃ and R₄, R₁₀ and R₁₁, R₁₁ and R₁₂, and R₁₂ and R₁₃ each independently form substituted or unsubstituted C3-C6 cycloalkane ring, substituted or unsubstituted 5-7 membered heterocycloalkane ring.

In another preferred embodiment, R₁, R₂, R₃, and R₄ are each independently hydrogen, substituted or unsubstituted C1-C6 alkyl, substituted or unsubstituted C1-C6 alkoxyl; or R₂ and R₃, R₁₀ and R₁₁, and R₁₁ and R₁₂ each independently form substituted or unsubstituted C3-C6 cycloalkane ring, substituted or unsubstituted 5-7 membered heterocycloalkane ring, wherein, the heterocycloalkane ring comprises 1-3 oxygen atoms.

In another preferred embodiment, the heterocycloalkane ring is substituted or unsubstituted 1,3-dioxole ring.

In another preferred embodiment, R₁ is hydrogen.

In another preferred embodiment, R₂ and R₃ form substituted or unsubstituted 3-membered heterocycloalkane ring, substituted or unsubstituted 4-membered heterocycloalkane ring, substituted or unsubstituted 5-membered heterocycloalkane ring, substituted or unsubstituted 6-membered heterocycloalkane ring, substituted or unsubstituted 7-membered heterocycloalkane ring, substituted or unsubstituted 8-membered heterocycloalkane ring, substituted or unsubstituted 9-membered heterocycloalkane ring, substituted or unsubstituted 10-membered heterocycloalkane ring, substituted or unsubstituted 11-membered heterocycloalkane ring, or substituted or unsubstituted 12-membered heterocycloalkane ring,

In another preferred embodiment, R₂ and R₃ form substituted or unsubstituted1, 3-dioxole ring.

In the present invention, the structure of the 1, 3-dioxole ring is as follows:

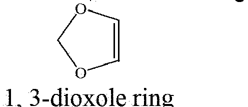

In another preferred embodiment, R₂ and R₃ form substituted or unsubstituted; 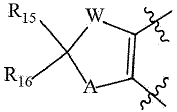
A is O or S;
W is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, A is O or S.

In another preferred embodiment, W is O or S.

In another preferred embodiment, R₂ and R₃ form substituted or unsubstituted 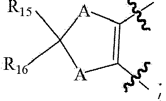
A is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₁ and R₂, R₂ and R₃, R₃ and R₄, R₁₀ and R₁₁, R₁₁ and R₁₂, and R₁₂ and R₁₃ each independently form 5-7 membered heterocycloalkane ring, preferably

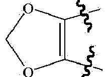

In another preferred embodiment, R₂ and R₃ form substituted or unsubstituted

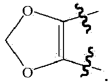

In another preferred embodiment, R₄ is hydrogen.

In another preferred embodiment, R₅, R₆, R₇, R₈, R₉ and R₁₄ are each independently hydrogen, halogen, - CN, hydroxyl, sulfhydryl, nitro, amino, -COOH, -CHO, substituted or unsubstituted C1-C8 alkyl, substituted or unsubstituted C3-C10 cycloalkyl, substituted or unsubstituted C1-C8 alkoxyl, substituted or unsubstituted C1-C8 alkylthio, substituted or unsubstituted 3-10 membered heterocycloalkyl, substituted or unsubstituted C6-C12 aryl, or substituted or unsubstituted 3-10 membered heteroaryl.

In another preferred embodiment, R₅, R₆, R₇, R₈, R₉ and R₁₄ are each independently hydrogen, halogen, - CN, hydroxyl, sulfhydryl, nitro, amino, -COOH, -CHO, substituted or unsubstituted C1-C6 alkyl, substituted or unsubstituted C3-C8 cycloalkyl, substituted or unsubstituted C1-C6 alkoxyl, substituted or unsubstituted C1-C6 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₅, R₆, R₇, R₈, R₉ and R₁₄ are each independently hydrogen, halogen, - CN, hydroxyl, sulfhydryl, nitro, amino, -COOH, -CHO, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₅, R₆, R₇, R₈ and R₉ are each independently hydrogen, methyl, ethyl, propyl, or butyl.

In another preferred embodiment, R₁₀, R₁₁ and R₁₂ are each independently hydrogen, substituted or unsubstituted C1-C6 alkoxyl, substituted or unsubstituted C1-C6 alkylthio; or R₁₀ and R₁₁, and R₁₁ and R₁₂ each independently form substituted or unsubstituted 3-membered heterocycloalkane ring, substituted or unsubstituted 4-membered heterocycloalkane ring, substituted or unsubstituted 5-membered heterocycloalkane ring, substituted or unsubstituted 6-membered heterocycloalkane ring, substituted or unsubstituted 7-membered heterocycloalkane ring, substituted or unsubstituted 8-membered heterocycloalkane ring, substituted or unsubstituted 9-membered heterocycloalkane ring, substituted or unsubstituted 10-membered heterocycloalkane ring, substituted or unsubstituted 11-membered heterocycloalkane ring, or substituted or unsubstituted 12-membered heterocycloalkane ring,

In another preferred embodiment, R₁₀, R₁₁ and R₁₂ are each independently hydrogen, substituted or unsubstituted C1-C4 alkoxyl, substituted or unsubstituted C1-C4 alkylthio; or R₁₀ and R₁₁, and R₁₁ and R₁₂ each independently form substituted or unsubstituted 3-membered heterocycloalkane ring, substituted or unsubstituted 4-membered heterocycloalkane ring, substituted or unsubstituted 5-membered heterocycloalkane ring, substituted or unsubstituted 6-membered heterocycloalkane ring, substituted or unsubstituted 7-membered heterocycloalkane ring, substituted or unsubstituted 8-membered heterocycloalkane ring, substituted or unsubstituted 9-membered heterocycloalkane ring, substituted or unsubstituted 10-membered heterocycloalkane ring, substituted or unsubstituted 11-membered heterocycloalkane ring, or substituted or unsubstituted 12-membered heterocycloalkane ring,

In another preferred embodiment, the heterocycloalkane ring is substituted or unsubstituted 1,3-dioxole ring.

In another preferred embodiment, R₁₀, R₁₁, and R₁₂ are each independently hydrogen, R₁₇-A-, A is O or S;

R₁₇ is substituted or unsubstituted C1-C10 alkyl, C2-C4 alkenyl-C1-C4 alkyl-, C2-C8 ester group-C1-C4 alkyl-, C1-C12 haloalkyl, 5-7 membered heterocycloalkyl-C1-C4 alkyl-, pyrrolidine-2,5-diketone group-C1-C4 alkyl-, cyano-C1-C4 alkyl-, C1-C4 alkoxyl-C2-C4 alkyl-, C2-C4 alkylthio-C1-C4 alkyl-, 5-7 membered heteroaryl-C1-C4 alkyl-.

In another preferred embodiment, R₁₇ is substituted or unsubstituted C3-C10 alkyl, C2-C4 alkenyl-C1-C4 alkyl-, C2-C8 ester group-C1-C4 alkyl-, C1-C12 haloalkyl, 5-7 membered heterocycloalkyl-C1-C4 alkyl-, pyrrolidine-2,5-diketone group-C1-C4 alkyl-, cyano-C1-C4 alkyl-, C1-C4 alkoxyl-C2-C4 alkyl-, C2-C4 alkylthio-C1-C4 alkyl-, 5-7 membered heteroaryl-C1-C4 alkyl-.

In another preferred embodiment, R₁₇ is substituted or unsubstituted C1-C6 alkyl, C2-C4 alkenyl-C1-C4 alkyl-, C2-C8 ester group-C1-C4 alkyl-, C1-C12 haloalkyl, 5-7 membered heterocycloalkyl-C1-C4 alkyl-, pyrrolidine-2,5-diketone group-C1-C4 alkyl-, cyano-C1-C4 alkyl-, C1-C4 alkoxyl-C2-C4 alkyl-, C2-C4 alkylthio-C1-C4 alkyl-, 5-7 membered heteroaryl-C1-C4 alkyl-.

In another preferred embodiment, R₁₇ is substituted or unsubstituted C3-C6 alkyl, C2-C4 alkenyl-C1-C4 alkyl-, C2-C8 ester group-C1-C4 alkyl-, C1-C12 haloalkyl, 5-7 membered heterocycloalkyl-C1-C4 alkyl-, pyrrolidine-2,5-diketone group-C1-C4 alkyl-, cyano-C1-C4 alkyl-, C1-C4 alkoxyl-C2-C4 alkyl-, C2-C4 alkylthio-C1-C4 alkyl-, 5-7 membered heteroaryl-C1-C4 alkyl-.

In another preferred embodiment, R₁₇ is substituted or unsubstituted C1-C6 alkyl, C2-C4 alkenyl-C1-C2 alkyl-, C2-C8 ester group-C1-C2 alkyl-.

In another preferred embodiment, R₁₇ is substituted or unsubstituted C1-C4 alkyl, C2-C4 alkenyl-C1-C2 alkyl-, C2-C8 ester group-C1-C2 alkyl-.

In another preferred embodiment, R₁₇ is substituted or unsubstituted C1-C4 alkyl, C2-C4 alkenyl-C1-C2 alkyl-, C3-C7 ester group-C1-C2 alkyl-.

In another preferred embodiment, R₁₇ is substituted or unsubstituted C3-C6 alkyl, C2-C4 alkenyl-C1-C2 alkyl-, C2-C8 ester group-C1-C2 alkyl-.

In another preferred embodiment, R₁₇ is methyl, ethyl, propyl, butyl, pentyl, hexyl, heptyl, octyl, nonyl, vinyl-methyl-, amyl ester group-methyl-, halopropyl, halodecyl, morpholinyl-ethyl-, pyrrolidine-2,5-diketo-ethyl-, cyano-methy -, methoxyl-ethyl-, pyridyl-methyl-.

In another preferred embodiment, amyl ester group is -COOC(CH₃)₃.

In another preferred embodiment, the halo is monohalop, dihalo, or trihalo.

In another preferred embodiment, the propyl is n-propyl.

In another preferred embodiment, the decyl is n-decyl.

In another preferred embodiment, the hexyl is n-hexyl.

In another preferred embodiment, R₁₀, R₁₁ and R₁₂ are each independently hydrogen, methoxyl, ethoxyl, propoxyl, butoxyl, methylthio, ethylthio, propylthio, or butythio.

In another preferred embodiment, R₁₀ and R₁₁, R₁₁ and R₁₂ each independently form substituted or unsubstituted 1, 3-dioxole ring.

In another preferred embodiment R₁₀ and R₁₁ form substituted or unsubstituted 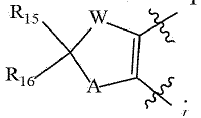
A is O or S;
W is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₁₀ and R₁₁ form substituted or unsubstituted [1, 3]-dioxole ring.

In another preferred embodiment, R₁₀ and R₁₁ form substituted or unsubstituted 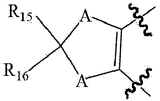
; A is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₁₀ and R₁₁ form substituted or unsubstituted

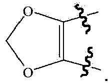

In another preferred embodiment R₁₁ and R₁₂ form substituted or unsubstituted 1,3-dioxole ring.

In another preferred embodiment, R₁₁ and R₁₂ form substituted or unsubstituted 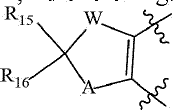
; A is O or S;
W is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, A is O or S.

In another preferred embodiment W is O or S.

In another preferred embodiment, R₁₁ and R₁₂ form substituted or unsubstituted 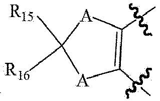 ;
A is O or S;
R₁₅ and R₁₆ are each independently hydrogen, substituted or unsubstituted C1-C4 alkyl, substituted or unsubstituted C3-C8 cycloalkyl, substituted or unsubstituted C1-C4 alkoxyl, substituted or unsubstituted C1-C4 alkylthio, substituted or unsubstituted 3-8 membered heterocycloalkyl, substituted or unsubstituted C6-C12 aryl, or substituted or unsubstituted 3-8 membered heteroaryl.

In another preferred embodiment, R₁₁ and R₁₂ form substituted or unsubstituted

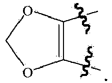

In another preferred embodiment, R₁₃ is hydrogen, substituted or unsubstituted C1-C6 alkoxyl, substituted or unsubstituted C1-C6 alkylthio.

In another preferred embodiment, R₁₃ is hydrogen.

In another preferred embodiment, R₁₄ is hydrogen, methyl, ethyl, propyl, or butyl.

In another preferred embodiment, R₁₄ is methyl.

In another preferred embodiment, the compound of formula I has the following structure of formula 1-1: 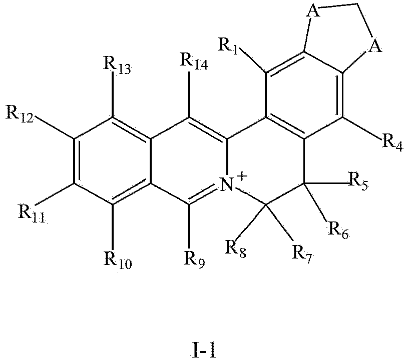 wherein,
A is O or S;
R₁, R₄, R₅, R₆, R₇, R₈, R₉, R₁₀, R₁₁, R₁₂, R₁₃ and R₁₄ are as defined above.

In another preferred embodiment, the compound of formula I has the following structure of 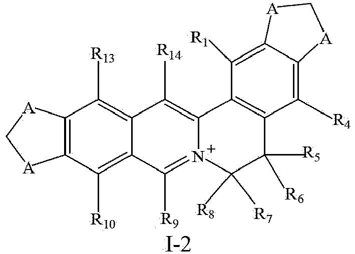 wherein,
A is O or S;
R₁, R₄, R₅, R₆, R₇, R₈, R₉, R₁₀, R₁₃ and R₁₄ are as defined above.

In another preferred embodiment, the compound of formula I has the following structure of formula 1-3: 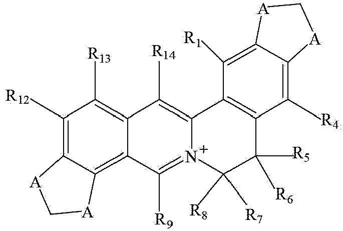 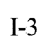 wherein,
A is O or S;
R₁, R₄, R₅, R₆, R₇, R₈, R₉, R₁₂, R₁₃ and R₁₄ are as defined above.

In another preferred embodiment, the compound of formula I has the following structure of formula 1-4: 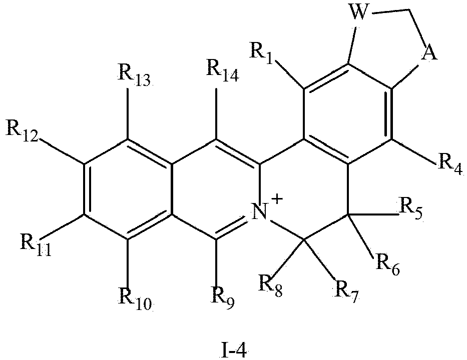 wherein,
A is O or S;
W is O or S;
R₁, R₄, R₅, R₆, R₇, R₈, R₉, R₁₀, R₁₁, R₁₂, R₁₃ and R₁₄ are as defined above.

In another preferred embodiment, the compound of formula I has the following structure of formula I-5: 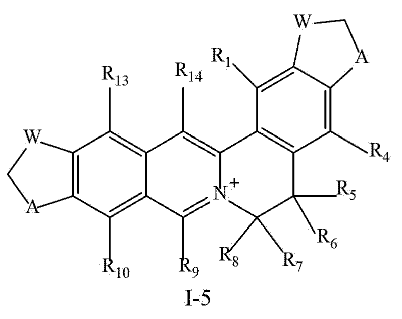 wherein,
A is O or S;
W is O or S;
R₁, R₄, R₅, R₆, R₇, R₈, R₉, R₁₀, R₁₃ and R₁₄ are as defined above.

In another preferred embodiment, the compound of formula I has the following structure of formula 1-6: 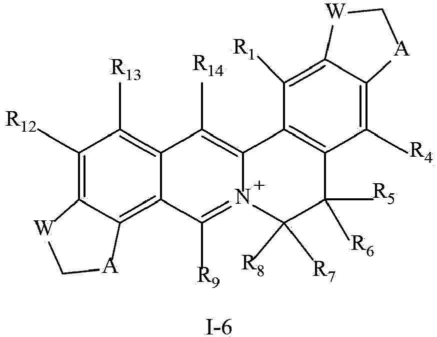 wherein,
A is O or S;
W is O or S;
R₁, R₄, R₅, R₆, R₇, R₈, R₉, R₁₂, R₁₃ and R₁₄ are as defined above.

In another preferred embodiment, the compound of formula I is selected from the following group:

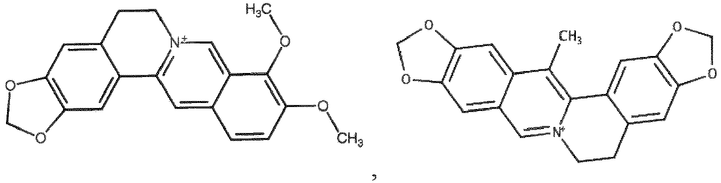

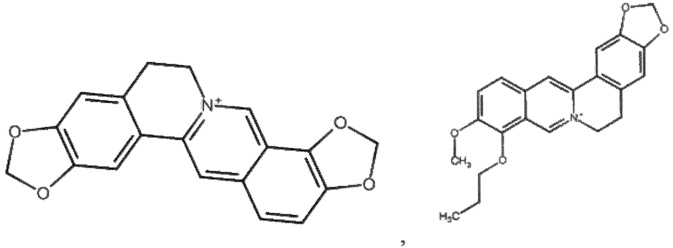

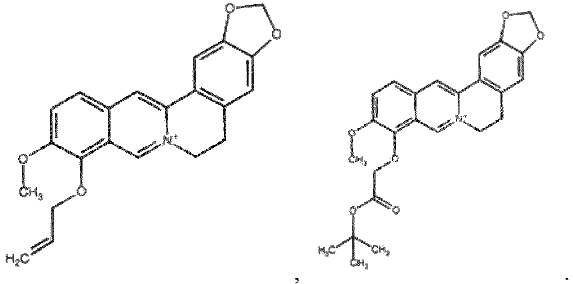

In another preferred embodiment, the compound of formula I is selected from the following group:

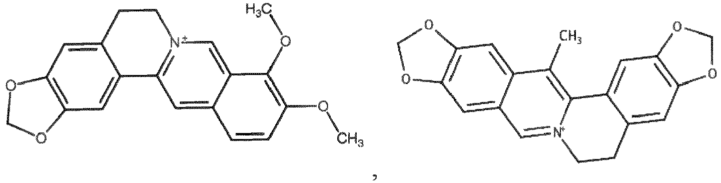

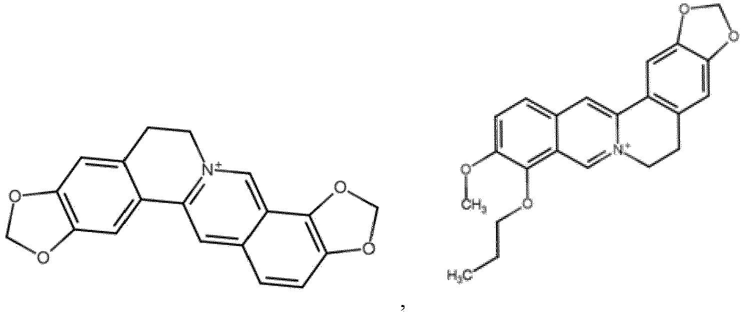

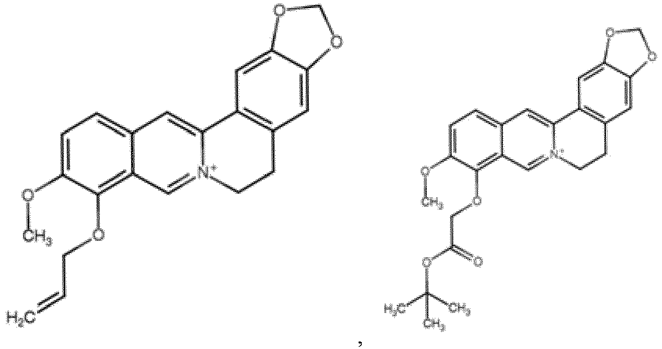

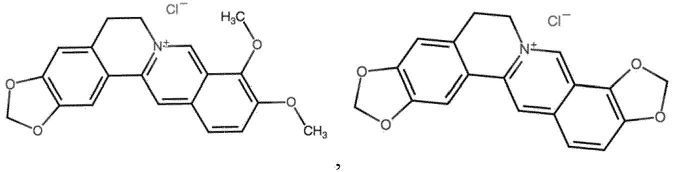

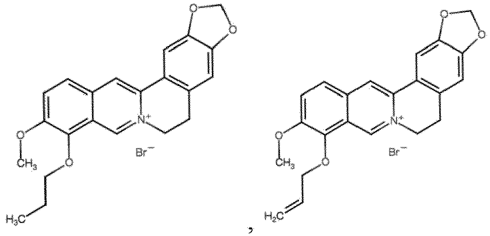

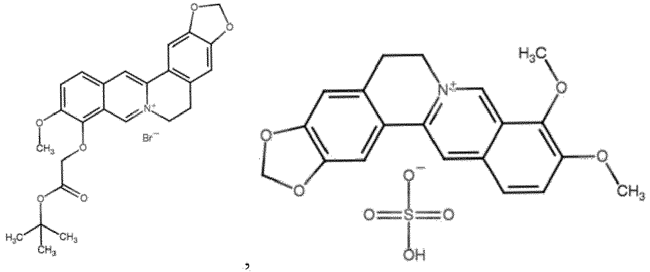

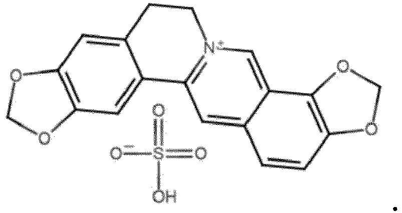

In another preferred embodiment, the compound of formula I comprises berberine, coptisine.

In another preferred embodiment, the berberine comprises berberine hydrochloride or berberine sulfate.

In another preferred embodiment, the coptisine comprises coptisine hydrochloride or coptisine sulfate.

In another preferred embodiment, the pharmaceutically acceptable salt of the compound of formula I is the salt formed by the compound of formula I and the acid selected from the group consisting of hydrochloric acid, mucic acid, D-glucuronic acid, hydrobromic acid, hydrofluoric acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid, glutamic acid, and combinations thereof, preferably hydrochloric acid, hydrobromic acid or sulfuric acid.

In another preferred embodiment, the tumor is human-derived tumor.

In another preferred embodiment, the tumor is human tumor.

In another preferred embodiment, the tumor comprises tumor with low or no expression of NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1 .

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRF1.

In another preferred embodiment, the tumor comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the tumor comprises tumor with high methylation level of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell (e.g., para-tumor tissue cell).

In another preferred embodiment, the tumor with low or no expression of NNMT gene means the ratio (E1/E0) of the expression level E1 of NNMT gene in the tumor cell to the expression level E0 of NNMT gene in the same type of cell or a normal cell (e.g., para-tumor tissue cell) is <1.0.

In another preferred embodiment, the low or no expression of NNMT gene means the ratio (E1/E0) of the expression level E1 of NNMT gene in a cell (e.g., tumor cell) to the expression level E0 of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the same type of cell refers to the cell ( e.g., the same type of tumor cell ) with normal expression of NNMT gene.

In another preferred embodiment, the same type of cell refers to the same type of cell with normal expression of NNMT gene.

In another preferred embodiment, the normal cell refers to normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal expression of NNMT gene.

In another preferred embodiment, the cell with normal expression of NNMT gene comprises the cell that is not sensitive to the compound of formula I, or an optical isomer thereof, or a racemate thereof, or a solvate thereof, or a pharmaceutically acceptable salt.

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same type of cell or a normal cell (e.g., para-tumor tissue cell).

In another preferred embodiment, the tumor with high expression of DNA methylase means the ratio (A1/A0) of the expression level A1 of DNA methylase in the tumor cell to the expression level A0 of DNA methylase in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell) with normal expression of DNA methylase.

In another preferred embodiment, the same type of cell refers to the same type of cell with normal expression of DNA methylase.

In another preferred embodiment, the normal cell refers to normal tissue cell (e.g., tumor origin cell, tumor-adjacent cell or para-tumor tissue cell) with normal expression of DNA methylase.

In another preferred embodiment, A0 refers to the expression level of DNA methylase in the cell with normal expression of DNA methylase.

In another preferred embodiment, the cell with normal expression of DNA methylase comprises the cell that is not sensitive to the isoquinoline compound.

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor cell is higher than that in the same type of cell or a normal cell (e.g., para-tumor tissue cell).

In another preferred embodiment, the tumor with high expression of DNMT1 means the ratio (B1/B0) of the expression level B1 of DNMT1 in the tumor cell to the expression level B0 of DNMT1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell) with normal expression of DNMT1.

In another preferred embodiment, the same type of cell refers to the same type of cell with normal expression of DNMT1.

In another preferred embodiment, the normal cell refers to normal tissue cell (e.g., tumor origin cell, tumor-adjacent cell or para-tumor tissue cell) with normal expression of DNMT1.

In another preferred embodiment, B0 refers to the expression level of DNMT1 in the cell with normal expression of DNMT1.

In another preferred embodiment, the cell with normal expression of DNMT1 comprises the cell that is not sensitive to the isoquinoline compound.

In another preferred embodiment, the tumor with high expression of DNMT3a means that DNMT3a protein can be detected in 20 µg of protein extracted from tumor using DNMT3a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same type of cell or a normal cell (e.g., para-tumor tissue cell).

In another preferred embodiment, the tumor with high expression of DNMT3a means the ratio (C1/C0) of the expression level C1 of DNMT3a in the tumor cell to the expression level C0 of DNMT3a in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably >_ 8, more preferably >_ 10, more preferably ≥ 15, more preferably >_ 20, more preferably ≥ 30, more preferably ≥ 50.

In another preferred embodiment, the same type of cell refers to the cell ( e.g., the same type of tumor cell) with normal expression of DNMT3a.

In another preferred embodiment, the same type of cell refers to the same type of cell with normal expression of DNMT3a.

In another preferred embodiment, the normal cell refers to normal tissue cell (e.g., tumor origin cell, tumor-adjacent cell or para-tumor tissue cell) with normal expression of DNMT3a.

In another preferred embodiment, C0 refers to the expression level of DNMT3a in the cell with normal expression of DNMT3a.

In another preferred embodiment, the cell with normal expression of DNMT3a comprises the cell that is not sensitive to the isoquinoline compound.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor cell is higher than that in the same type of cell or a normal cell (e.g., para-tumor tissue cell).

In another preferred embodiment, the tumor with high expression of DNMT3b means the ratio (D1/D0) of the expression level D1 of DNMT3b in the tumor cell to the expression level D0 of DNMT3b in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably >_ 10, more preferably >_ 15, more preferably ≥ 20, more preferably ≥ 30, more preferably ≥ 50.

In another preferred embodiment, the same type of cell refers to the cell ( e.g., the same type of tumor cell ) with normal expression of DNMT3b.

In another preferred embodiment, the same type of cell refers to the same type of cell with normal expression of DNMT3b.

In another preferred embodiment, the normal cell refers to normal tissue cell (e.g., tumor origin cell, tumor-adjacent cell or para-tumor tissue cell) with normal expression of DNMT3b

In another preferred embodiment, D0 refers to the expression level of DNMT3b in the cell with normal expression of DNMT3b.

In another preferred embodiment, the cell with normal expression of DNMT3b comprises the cell that is not sensitive to the isoquinoline compound.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF1 means the expression level of UHRF1 in tumor cell is higher than that in the same type of cell or a normal cell (e.g., para-tumor tissue cell).

In another preferred embodiment, the tumor with high expression of UHRF1 means the ratio (F1/F0) of the expression level F1 of UHRF1 in the tumor cell to the expression level F0 of UHRF1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of UHRF1.

In another preferred embodiment, the same type of cell refers to the same type of cell with normal expression of UHRF 1.

In another preferred embodiment, the normal cell refers to normal tissue cell (e.g., tumor origin cell, tumor-adjacent cell or para-tumor tissue cell) with normal expression of UHRF 1

In another preferred embodiment, F0 refers to the expression level of UHRF 1 in the cell with normal expression of UHRF1.

In another preferred embodiment, the cell with normal expression of UHRF1 comprises the cell that is not sensitive to the isoquinoline compound.

In another preferred embodiment, the high methylation level of nucleotide site of NNMT gene means the methylation level of nucleotide site of NNMT gene in a cell ( e.g., tumor cell) is higher than that in the same type of cell or a normal cell (e.g., para-tumor tissue cell).

In another preferred embodiment, the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of nucleotide site of NNMT gene means the methylation level of nucleotide site of NNMT gene in a cell ( e.g., tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of nucleotide site of NNMT gene.

In another preferred embodiment, the same type of cell refers to the same type of cell with normal methylation level of nucleotide site of NNMT gene.

In another preferred embodiment, the normal cell refers to normal tissue cell (e.g., tumor origin cell, tumor-adjacent cell or para-tumor tissue cell) with normal methylation level of nucleotide site of NNMT gene.

another preferred embodiment, the cell with normal methylation level of nucleotide site of NNMT gene comprises the cell that is not sensitive to the compound of formula I, or an optical isomer thereof, or a racemate thereof, or a solvate thereof, or a pharmaceutically acceptable salt.

In another preferred embodiment, the high methylation level of nucleotide site of NNMT gene means the methylation level (M%) of nucleotide site of NNMT gene in a cell ( e.g., tumor cell ) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell ( e.g., tumor cell) is higher than that in the same type of cell or a normal cell (e.g., para-tumor tissue cell).

In another preferred embodiment, the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of DNA CpG site of NNMT gene means the methylation level of DNA CpG site of NNMT gene in a cell ( e.g., tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of DNA CpG site of NNMT gene.

In another preferred embodiment, the same type of cell refers to the same type of cell with normal methylation level of DNA CpG site of NNMT gene.

In another preferred embodiment, the normal cell refers to normal tissue cell (e.g., tumor origin cell, tumor-adjacent cell or para-tumor tissue cell) with normal methylation level of DNA CpG site of NNMT gene.

In another preferred embodiment, the cell with normal methylation level of DNA CpG site of NNMT gene comprises the cell that is not sensitive to the compound of formula I, or an optical isomer thereof, or a racemate thereof, or a solvate thereof, or a pharmaceutically acceptable salt.

In another preferred embodiment, the high methylation level of DNA CpG site of NNMT gene means the methylation level (M%) of DNA CpG site of NNMT gene in a cell ( e.g., tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated CpG sites to the number of all CpG sites in the NNMT gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of nucleotide site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of the nucleotide site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the tumor is selected from the group consisting of lung cancer, renal carcinoma, breast cancer, colon cancer, lymphoma, leukemia, pancreatic cancer, brain tumor, liver cancer, prostate cancer, and combinations thereof.

In another preferred embodiment, the lung cancer is selected from the group consisting of non-small cell lung cancer, small cell lung cancer, and combinations thereof.

In another preferred embodiment, the colon cancer comprises colon adenocarcinoma.

In another preferred embodiment, the lymphoma is selected from the group consisting of B-cell lymphoma, skin T-cell lymphoma, and combinations thereof.

In another preferred embodiment, the lymphoma comprises diffuse large B-cell lymphoma.

In another preferred embodiment, the brain tumor is selected from the group consisting of glioblastoma, neuroglioma, brain medulloblastoma, brain neuroblastoma, and combination thereof.

In another preferred embodiment, the brain medulloblastoma comprises cerebellar medulloblastoma.

In another preferred embodiment, the glioblastoma comprises glioblastoma multiforme.

In another preferred embodiment, the brain tumor comprises glioblastoma.

In another preferred embodiment, the renal carcinoma is selected from the group consisting of clear cell renal cell adenocarcinoma, renal carcinoma Wilms, and combination thereof.

In another preferred embodiment, the renal carcinoma comprises clear cell renal cell adenocarcinoma.

In another preferred embodiment, the renal carcinoma comprises renal carcinoma Wilms.

In another preferred embodiment, the renal carcinoma cell comprises renal carcinoma Wilms cell.

In another preferred embodiment, the leukemia is selected from the group consisting of T-lymphocyte leukemia, myeloid leukemia, and combinations thereof.

In another preferred embodiment, the T-lymphocytic leukemia comprises acute T-lymphocytic leukemia.

In another preferred embodiment, the myeloid leukemia comprises type M4 of acute myeloid leukemia.

In another preferred embodiment, the myeloid leukemia comprises FAB type M4 of acute myeloid leukemia.

In another preferred embodiment, the expression comprises protein expression and/or mRNA expression.

In another preferred embodiment, the composition is a pharmaceutical composition.

In another preferred embodiment, the composition or preparation further comprises a pharmaceutically acceptable carrier.

In another preferred embodiment, the expression is mRNA expression or protein expression.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation

In another preferred embodiment, the dosage form of the composition or preparation is tablet, injection, infusion, paste, gel, solution, microsphere or film.

In the second aspect of the present invention, it provides a marker for determining whether the compound of formula I, or an optical isomer thereof, or a racemate thereof, or a solvate thereof, or a pharmaceutically acceptable salt according to the first aspect of the present invention is suitable for use in the prevention and/or treatment of patient tumor, the marker comprises NNMT gene, and/or the methylation level of DNA CpG site of NNMT gene.

In another preferred embodiment, the marker comprises the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the marker comprises the NNMT gene, DNA methylase, UHRF1, nucleotide site of NNMT gene, and/or DNA CpG site of NNMT gene.

In another preferred embodiment, the compound of formula I, or an optical isomer thereof, or a racemate thereof, or a solvate thereof, or a pharmaceutically acceptable salt according to the first aspect of the present invention is suitable for use in the prevention and/or treatment of patient tumor with low or no expression of NNMT gene, high expression of DNA methylase, high expression of UHRF1, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt according to the first aspect of the present invention is not suitable for use in the prevention and/or treatment of patient tumor with high expression of NNMT gene, low expression of DNA methylase, low expression of UHRF1, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt according to the first aspect of the present invention is suitable for use in the prevention and/or treatment of patient tumor" comprises "the patient tumor is sensitive to the compound of formula I, or an optical isomer thereof, or a racemate thereof, or a solvate thereof, or a pharmaceutically acceptable salt according to the first aspect of the present invention".

In another preferred embodiment, "the compound of formula I, or an optical isomer thereof, or a racemate thereof, or a solvate thereof, or a pharmaceutically acceptable salt according to the first aspect of the present invention is not suitable for use in the prevention and/or treatment of patient tumor" comprises "the patient tumor is not sensitive to the compound of formula I, or an optical isomer thereof, or a racemate thereof, or a solvate thereof, or a pharmaceutically acceptable salt according to the first aspect of the present invention".

In another preferred embodiment, the tumor with low or no expression of NNMT gene is as described in the first aspect of the invention.

In another preferred embodiment, the tumor with high expression of DNA methylase (e.g., DNMT1) is as described in the first aspect of the invention.

In another preferred embodiment, the tumor with high expression of UHRF1 is as described in the first aspect of the invention.

In another preferred embodiment, the tumor with high methylation level of nucleotide site of NNMT gene is as described in the first aspect of the invention.

In another preferred embodiment, the tumor with high methylation level of DNA CpG site of NNMT gene is as described in the first aspect of the invention.

In another preferred embodiment, the high expression of NNMT gene means the ratio (E1/E0) of the expression level E1 of NNMT gene in a cell ( e.g., tumor cell) to the expression level E0 of NNMT gene in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50.

In another preferred embodiment, the tumor with low expression of DNA methylase means the ratio (A1/A0) of the expression level A1 of DNA methylase in the tumor cell to the expression level A0 of DNA methylase in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably < 0.0001, more preferably ≤ 0.00001, more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell ( e.g., tumor cell) to the methylation level L0 of nucleotide site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In the third aspect of the present invention, it provides a detection kit, which comprises:
(i) a detection reagent for detecting the expression level of NNMT gene and/or the methylation level of DNA CpG site of NNMT gene.

In another preferred embodiment, the detection kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the expression of NNMT gene is the expression of mRNA and/or protein.

In another preferred embodiment, the methylation level of DNA CpG site of NNMT gene is the methylation level of DNA CpG site in promoter region of NNMT gene.

In another preferred embodiment, the methylation level of DNA CpG site of NNMT gene is the methylation level of DNA CpG sites from 1050 bp before the transcription start site to 499 bp after the transcription start site in NNMT gene.

In another preferred embodiment, the methylation level of DNA CpG site of NNMT gene is the methylation level of DNA CpG sites from 1050 bp to 193 bp before the transcription start site in NNMT gene.

In another preferred embodiment, the methylation level of DNA CpG site of NNMT gene is the methylation level of DNA CpG sites from 840 bp to 469 bp before the transcription start site in NNMT gene.

In the fourth aspect of the present invention, it provides a use of the detection kit according to the third aspect of the present invention in the preparation of concomitant diagnose kit for determining whether the compound of formula I, or an optical isomer thereof, or a racemate thereof, or a solvate thereof, or a pharmaceutically acceptable salt according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt according to the first aspect of the present invention is suitable for use in the prevention and/or treatment of patient tumor with low or no expression of NNMT gene, high expression of DNA methylase, high expression of UHRF1, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt according to the first aspect of the present invention is not suitable for use in the prevention and/or treatment of patient tumor with high expression of NNMT gene, low expression of DNA methylase, low expression of UHRF1, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt according to the first aspect of the present invention is suitable for use in the prevention and/or treatment of patient tumor" is as described in the second aspect of the invention.

In another preferred embodiment, "the compound of formula I, or an optical isomer thereof, or a racemate thereof, or a solvate thereof, or a pharmaceutically acceptable salt according to the first aspect of the present invention is not suitable for use in the prevention and/or treatment of patient tumor" is as described in the second aspect of the invention.

In the fifth aspect of the present invention, it provides a medicine kit, which comprises:
(i) a detection reagent for detecting the expression level of NNMT gene and/or the methylation level of DNA CpG site of NNMT gene; and
(ii) a compound of formula I, or an optical isomer thereof, or a racemate thereof, or a solvate thereof, or a pharmaceutically acceptable salt according to the first aspect of the present invention.

In another preferred embodiment, the medicine kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt according to the first aspect of the present invention.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt according to the first aspect of the present invention is suitable for use in the prevention and/or treatment of patient tumor with low or no expression of NNMT gene, high expression of DNA methylase, high expression of UHRF1, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt according to the first aspect of the present invention is not suitable for use in the prevention and/or treatment of patient tumor with high expression of NNMT gene, low expression of DNA methylase, low expression of UHRF1, low methylation level of nucleotide site of NNMT gene, and/or low methylation level of DNA CpG site of NNMT gene.

In the sixth aspect of the present invention, it provides a method for preventing and/or treating tumor, which comprises administering the compound of formula I, or an optical isomer thereof, or a racemate thereof, or a solvate thereof, or a pharmaceutically acceptable salt according to the first aspect of the present invention to a subject in need.

In another preferred embodiment, the tumor is as described in the first aspect of the invention.

In another preferred embodiment, the tumor of the subject comprises tumor with low or no expression of NNMT gene.

In another preferred embodiment, the tumor of the subject comprises tumor with high expression of DNA methylase.

In another preferred embodiment, the tumor of the subject comprises tumor with high expression of UHRF1.

In another preferred embodiment, the tumor of the subject comprises tumor with high methylation level of nucleotide site of NNMT gene.

In another preferred embodiment, the tumor of the subject comprises tumor with high methylation level of DNA CpG site of NNMT gene.

In the seventh aspect of the present invention, it provides a device or system, the device or system comprises:
(i) a detection module, the detection module is used to detect the expression level of NNMT gene, the expression level of DNA methylase, the expression level of UHRF1, the methylation level of nucleotide site of NNMT gene, and/or the methylation level of DNA CpG site of NNMT gene;
(ii) a output module, the output module comprises the output of the information as follows:
   the compound of formula I, or an optical isomer thereof, or a racemate thereof, or a solvate thereof, or a pharmaceutically acceptable salt according to the first aspect of the present invention is suitable for use in the prevention and/or treatment of patient tumor with low or no expression of NNMT gene, high expression of DNA methylase, high expression of UHRF1, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt according to the first aspect of the present invention is not suitable for use in the prevention and/or treatment of patient tumor with high expression of NNMT gene, low expression of DNA methylase, low expression of UHRF1, low methylation level of nucleotide site of NNMT gene, and/or low methylation level of DNA CpG site of NNMT gene.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In another preferred embodiment, the expression level of NNMT gene and/or the methylation level of DNA CpG site in promoter region of NNMT gene can be obtained by the procession of the data processing module.

In another preferred embodiment, the expression level of NNMT gene and/or the methylation level of DNA CpG sites from 1050 bp before the transcription start site to 499 bp after the transcription start site in NNMT gene can be obtained by the procession of the data processing module.

In another preferred embodiment, the expression level of NNMT gene and/or the methylation level of DNA CpG sites from 1050 bp to 193 bp before the transcription start site in NNMT gene can be obtained by the procession of the data processing module.

In another preferred embodiment, the expression level of NNMT gene and/or the methylation level of DNA CpG site from 840 bp to 469 bp before the transcription start site in NNMT gene can be obtained by the procession of the data processing module.

In the eight aspect of the present invention, it provides a compound of formula I, or an optical isomer thereof, or a racemate thereof, or a solvate thereof, or a pharmaceutically acceptable salt thereof; 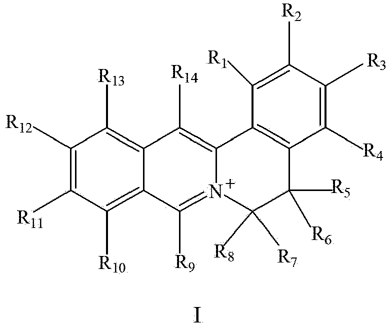 wherein,
R₁, R₂, R₃, R₄, R₅, R₆, R₇, R₈, R₉, R₁₀, R₁₁, R₁₂, R₁₃ and R₁₄ are as described in the first aspect of the invention.

In another preferred embodiment, the compound of formula I is as described in the first aspect of the invention.

In another preferred embodiment, the compound of formula I has the following structure of formula I-4; 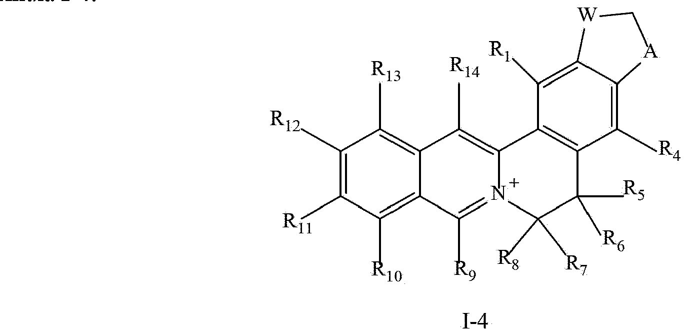 wherein,
A is O or S;
W is O or S;
R₁, R₄, R₅, R₆, R₇, R₈, R₉, R₁₀, R₁₁, R₁₂, R₁₃ and R₁₄ are as described in the first aspect of the invention.

In another preferred embodiment, R₁, R₄, R₁₀, R₁₁, R₁₂ and R₁₃ are each independently hydrogen, substituted or unsubstituted C1-C6 alkyl, substituted or unsubstituted C1-C6 alkoxyl, substituted or unsubstituted C1-C6 alkylthio.

In another preferred embodiment, R₅, R₆, R₇, R₈, R₉ and R₁₄ are hydrogen.

In another preferred embodiment, R₁₀ is hydrogen, R₁₇-A-,
A is O or S;
R₁₇ is substituted or unsubstituted C3-C6 alkyl, C2-C4 alkenyl-C1-C2 alkyl-, C2-C8 ester group-C1-C2 alkyl-.

In another preferred embodiment, the compound of formula I has the following structure of formula 1-1: 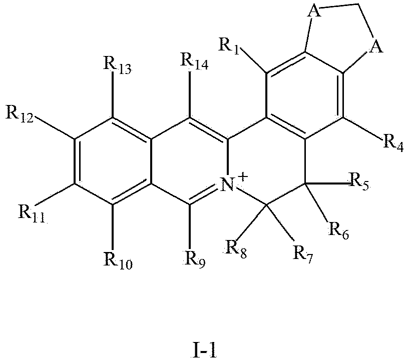 wherein,
A is O or S;
R₁, R₄, R₅, R₆, R₇, R₈, R₉, R₁₀, R₁₁, R₁₂, R₁₃ and R₁₄ are as described in the first aspect of the invention.

In another preferred embodiment, R₁₀ is hydrogen, R₁₇-A-,
A is O or S;
R₁₇ is substituted or unsubstituted C3-C6 alkyl, C2-C4 alkenyl-C1-C2 alkyl-, C2-C8 ester group-C1-C2 alkyl-.

In another preferred embodiment, the compound of formula I is selected from the following group:

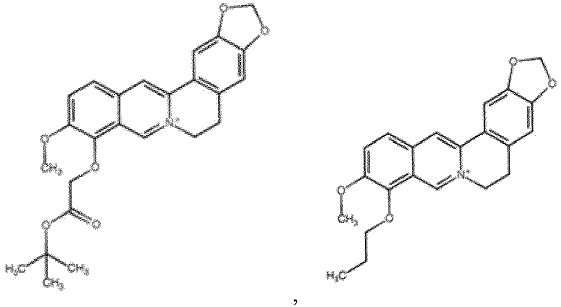

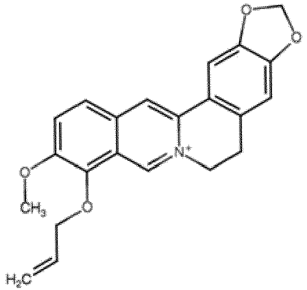

In another preferred embodiment, the compound of formula I is selected from the following group:

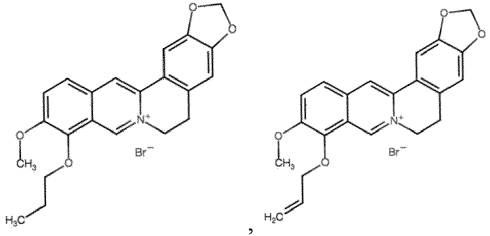

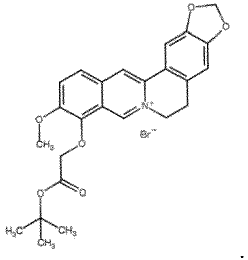

In the ninth aspect of the present invention, it provides a pharmaceutical composition, the pharmaceutical composition comprises the compound of formula I, or an optical isomer thereof, or a racemate thereof, or a solvate thereof, or a pharmaceutically acceptable salt thereof according to the eight aspect of the present invention.

It should be understood that, in the present invention, each of the technical features specifically described above and below (such as those in the Examples) can be combined with each other, thereby constituting new or preferred technical solutions which need not be redundantly described one-by-one.

### DESCRIPTION OF THE DRAWINGS

Fig. 1 shows the expression of NNMT gene in tumor cells sensitive and insensitive to isoquinoline compounds.
Fig.2 shows the methylation level of DNA CpG site of promoter region of NNMT gene in tumor cells sensitive and insensitive to isoquinoline compounds.
Fig.3 shows the methylation level of DNA CpG sites from 1050 bp before the transcription start site to 499 bp after the transcription start site in NNMT gene in tumor cells sensitive and insensitive to isoquinoline compounds.
Fig.4 shows the methylation level of DNA CpG sites from 1050 bp to 193 bp before the transcription start site in NNMT gene in tumor cells sensitive and insensitive to isoquinoline compounds.
Fig.5 shows the methylation level of DNA CpG sites of specific NNMT gene (ie, site 114165695, site 114165730, site 114165769, site 114165804, site 114165938, site 114166050, site 114166066 on human chromosome 11) in tumor cells sensitive and insensitive to isoquinoline compounds, black dot indicates that the relevant site is methylated, white dot indicates that the relevant site is not methylated, SST refers to the transcription starting site, and Chr11 refers to human chromosome 11 according to human genome version GCF_00000 1405.25 (GRCh37. p13).
Fig.6 shows the expression of NNMT gene in brain tumor cells sensitive and insensitive to isoquinoline compounds.
Fig.7 shows the methylation level of DNA CpG site of promoter region of NNMT gene in brain tumor cells sensitive and insensitive to isoquinoline compounds.
Fig.8 shows the methylation level of DNA CpG sites from 1050 bp before the transcription start site to 499 bp after the transcription start site in NNMT gene in brain tumor cells sensitive and insensitive to isoquinoline compounds.
Fig.9 shows the methylation level of DNA CpG sites from 1050 bp to 193 bp before the transcription start site in NNMT gene in brain tumor cells sensitive and insensitive to isoquinoline compounds.
Fig.10 shows the correlation between the expression of NNMT and the expression of DNMT1, UHRF1, DNMT3a and DNMT3b in tumor cells.

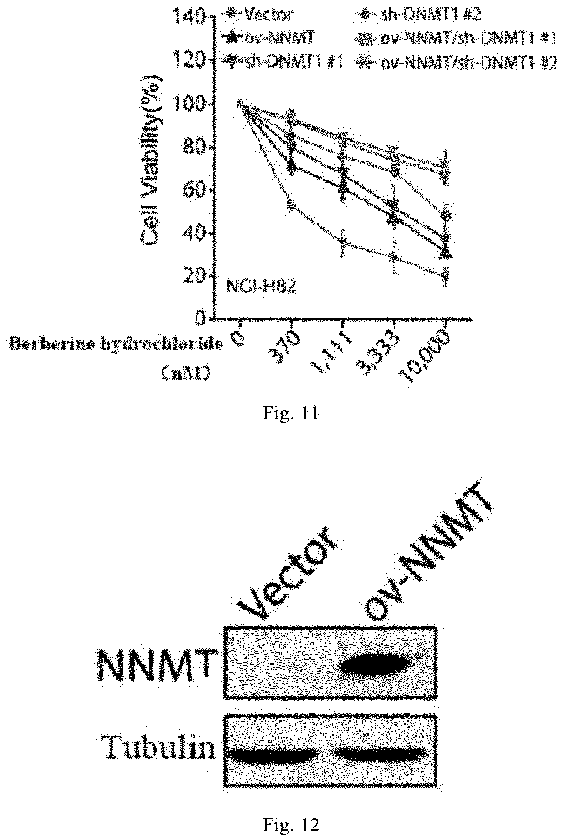

Fig.11 shows the sensitivity of tumor cells to berberine hydrochloride after overexpressing NNMT protein of NCI-H82 cell using transgenic method and/or knocking down DNMT1 expression of NCI-H82 cell using shRNA transfection method, wherein, "Vector" refers to NCI-H82 cell with normal expression of NNMT protein and DNMT1; "ov-NNMT" refers to NCI-H82 cell with overexpression of NNMT protein using transgenic method; "sh-DNMT1 # 1" refers to the NCI-H82 cell with knockdown of DNMT1 expression using sh-DNMT1#1 transfection method; "sh-DNMT1l#2" refers to the NCI-H82 cell with knockdown of DNMT1 expression using sh-DNMT1#2 transfection method; "ov-NNMT/sh-DNMT1#1" refers to NCI-H82 cell with overexpression of NNMT protein using transgenic method and knockdown of DNMT1 expression using sh-DNMT1#1 transfection method; "ov-NNMT/sh-DNMT1 #2" refers to NCI-H82 cell with overexpression of NNMT protein using transgenic method and knockdown of DNMT1 expression using sh-DNMT1#2 transfection method.
Fig.12 shows the NNMT protein content in NCI-H82 (ov-NNMT) overexpressing NNMT protein using Western Blot test compared with normal NCI-H82 (Vector), wherein, "Vector" refers to NCI-H82 cell with normal expression of NNMT protein; "ov-NNMT" refers to NCI-H82 cell with overexpression of NNMT protein using transgenic method.
Fig.13 shows the DNMT1 protein content in NCI-H82 (sh-DNMT1#1 or sh-DNMT1 # 2) with knockdown of DNMT1 expression using sh-DNMT1 # 1 or sh-DNMT1 # 2 transfection method via Western Blot test compared with normal NCI-H82 (shVector), wherein, "shVector" refers to NCI-H82 cell with normal expression of DNMT1 protein; "sh-DNMT1 # 1" refers to NCI-H82 cell with knockdown of DNMT1 expression using sh-DNMT1 # 1 transfection method; "sh-DNMT1 # 2" refers to NCI-H82 cell with knockdown of DNMT1 expression using sh-DNMT1 # 2 transfection method.
Fig.14 shows the inhibitory effect of berberine hydrochloride on NCI-H82 tumor in mice, wherein NCI-H82 refers to the NCI-H82 cell with normal expression of NNMT.
Fig.15 shows the inhibitory effect of berberine hydrochloride on NCI-H82 tumor (ov-NNMT/sh-DNMT1) with overexpressing of NNMT protein and low expression of DNMT1 in mice, wherein NCI-H82 ov-NNMT/sh-DNMT1 refers to NCI-H82 cell with overexpressing of NNMT protein and low expression of DNMT1.

### EMBODIMENTS FOR CARRYING OUT THE INVENTION

Based on an extensive and intensive research, the inventors have unexpectedly found the compound of present invention has significant inhibitory effect on tumor with low or no expression of NNMT gene, high expression of DNA methylase, high expression of UHRF1, high methylation level of nucleotide site of NNMT gene, and/or high methylation level of DNA CpG site of NNMT gene. The expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of present invention is suitable for use in the prevention and/or treatment of patient tumor. On this basis, the inventors has completed the present invention.

### Terms

As used herein, the term "comprise", " comprising", and "containing" are used interchangeably, which not only comprise closed definitions, but also semi-closed and open definitions. In other words, the term comprises "consisting of' and "essentially consisting of".

As used herein, the term "high methylation level of DNA CpG site" and"high level of DNA CpG site methylation" are used interchangeably.

As used herein, the term "low methylation level of DNA CpG site" and"low level of DNA CpG site methylation" are used interchangeably.

As used herein, the term "IC50" and "IC₅₀" are used interchangeably, and refers to 50% inhibiting concentration, ie, the concentration of the inhibitor when 50% inhibitory effect is achieved.

As used herein, the term "methylation of CpG site", "methylation of CpG nucleotide" and "CpG methylation" are used interchangeably.

As used herein, the term "P/S" refers to adding penicillin and streptomycin into the culture medium.

As used herein, the term "a cell" refers to a cell (e.g., single tumor cell) or a group of cells containing multiple similar cells ((e.g., a tumor tissue).

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, "the expression level of NNMT gene, the expression level of DNA methylase, the expression level of UHRF1, the methylation level of nucleotide site of NNMT gene, and/or the methylation level of DNA CpG site of NNMT gene" refers to one or more of the expression level of NNMT gene, the expression level of DNA methylase, the expression level of UHRF1, the methylation level of nucleotide site of NNMT gene, and the methylation level of DNA CpG site of NNMT gene.

As used herein, "the low or no expression of NNMT gene, high expression of DNA methylase, high expression of UHRF1, high methylation level of nucleotide site of NNMT gene, and/or high methylation level of DNA CpG site of NNMT gene" refers to one or more of the low or no expression of NNMT gene, high expression of DNA methylase, high expression of UHRF1, high methylation level of nucleotide site of NNMT gene, and high methylation level of DNA CpG site of NNMT gene.

As used herein, "the high expression of NNMT gene, low expression of DNA methylase, low expression of UHRF1, low methylation level of nucleotide site of NNMT gene, and/or low methylation level of DNA CpG site of NNMT gene" refers to one or more of the high expression of NNMT gene, low expression of DNA methylase, low expression of UHRF1, low methylation level of nucleotide site of NNMT gene, and low methylation level of DNA CpG site of NNMT gene.

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_000001405.25 (GRCh37. p13).

As used herein, the term "human chromosome 11" refers to human chromosome 11 according to human genome version GCF_ 000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide of site 114165695 on human chromosome 11; "site 114165730 on human chromosome 11" refers to nucleotide of site 114165730 on human chromosome 11; "site 114165769 on human chromosome 11" refers to nucleotide of site 114165769 on human chromosome 11; "site 114165804 on human chromosome 11" refers to nucleotide of site 114165804 on human chromosome 11; "site 114165938 on human chromosome 11" refers to nucleotide of site 114165938 on human chromosome 11; "site 114166050 on human chromosome 11" refers to nucleotide of site 114166050 on human chromosome 11; "site 114166066 on human chromosome 11" refers to nucleotide of site 114166066 on human chromosome 11.

As used herein, the gene expression comprises the protein expression of the gene and/or the mRNA expression of the gene.

As used herein, the term "DNMT3a" refers to DNA methyltransferase 3a.

As used herein, the term "DNMT3b" refers to DNA methyltransferase 3b.

As used herein, the term "DNMT1" refers to DNA methyltransferase 1.

As used herein, the term "UHRF 1" refers to ubiquitin-like with PHD and ring finger domain 1.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc.

As used herein, "R₁", "R1" and "R¹" have the same meaning and can be used interchangeably. The other similar definitions have the same meaning.

As used herein, " 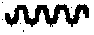 " denotes the linking site of the group.

As used herein, the term "alkyl" refers to a saturated hydrocarbon group with linear chain (ie, unbranched chain) or branched chain, or a combination of linear and branched chains. When the number of carbon atoms is limited in front of the alkyl (e.g., C1-C6 alkyl), it means that the alkyl has 1-6 carbon atoms, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "C2-C4 alkenyl" refers to a hydrocarbon group formed by the loss of one hydrogen atom connected to the double bond in linear or branched alkene with 2-4 carbon atoms and one or more double bonds, such as CH₂=CH-, C(CH₃)₂=CH-, or the like.

As used herein, the term "C2-C4 alkynyl" refers to a hydrocarbon group formed by the loss of one hydrogen atom connected to the triple bond in linear or branched alkyne with 2-4 carbon atoms and one or more double bonds, such as (CH≡CH-), (H₃C-C≡CH-), or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the haloalkyl (e.g., C1-C8 haloalkyl), it means that the haloalkyl has 1-8 carbon atoms, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkane ring" refers to a cyclic ring having a saturated or partially saturated monocyclic ring, bicyclic ring or polycyclic ring (fused ring, bridged ring or spiro ring). When the number of carbon atoms is limited in front of the cycloalkane ring (e.g., C3-C12 cycloalkane ring), it means the cycloalkane ring has 3-12 ring carbon atoms. In some preferred embodiments, C3-C8 cycloalkane ring refers to a saturated or partially saturated monocycloalkane ring or dicycloalkane ring having 3-8 ring carbon atoms, comprising cyclopropane ring, cyclobutane ring, cyclopentane ring, cycloheptane ring, or the like. The term "spirocycloalkane ring" refers to a bicyclic or polycyclic ring that shares a carbon atom (referred to spiro-atom) between single rings, the spirocycloalkane ring can contain one or more double bonds, but no ring has a fully conjugated π electron system. Fused cycloalkane ring refers to all carbon bicyclic or polycyclic rings in which each ring in the system shares an adjacent pair of carbon atoms with other rings, one or more rings can contain one or more double bonds, but no ring has a fully conjugated π electron system. Bridged cycloalkane ring refers to all carbon polycyclic ring in which any two rings share two non-directly connected carbon atoms, the bridged cycloalkane ring can contain one or more double bonds, but no ring has a fully conjugated π electron system. The representative examples of cycloalkane ring comprise but are not limited to cyclopropane ring, cyclobutane ring, cyclopentane ring, cycloheptane ring, or the like.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it means the cycloalkyl has 3-12 ring carbon atoms. In some preferred embodiments, C3-C8 cycloalkyl refers to a saturated or partially saturated monocycloalkyl or dicycloalkyl having 3-8 ring carbon atoms, comprising cyclopropyl, cyclobutyl, cyclopentane , cycloheptyl, or the like. The term "spirocycloalkyl" refers to a bicyclic or polycyclic group that shares a carbon atom (referred to spiro-atom) between single rings, the spirocycloalkyl can contain one or more double bonds, but no ring has a fully conjugated π electron system. Fused cycloalkyl refers to all carbon bicyclic or polycyclic groups in which each ring in the system shares an adjacent pair of carbon atoms with other rings, one or more rings can contain one or more double bonds, but no ring has a fully conjugated π electron system. Bridged cycloalkyl refers to all carbon polycyclic group in which any two rings share two non-directly connected carbon atoms, the bridged cycloalkyl can contain one or more double bonds, but no ring has a fully conjugated π electron system. The representative examples of cycloalkyl comprise but are not limited to

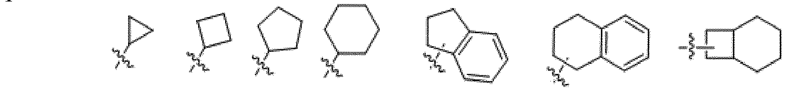

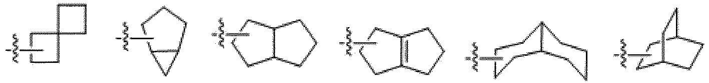

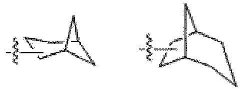

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the halocycloalkyl (e.g, C3-C8 haloalkyl), it means that the halocycloalkyl has 3-8 ring carbon atoms, for example, C3-C8 haloalkyl refers to an halocycloalkyl having 3-6 carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-S- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "cycloalkoxyl" refers to R-O- group, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 carbon atoms. Representative examples of cycloalkoxyl comprise but are not limited to cyclopropyloxyl, cyclobutoxyl, or the like.

As used herein, the term "cycloalkylthio" refers to R-S- group, wherein R is cycloalkyl, the cycloalkyl is as defined above. When the number of carbon atoms is limited in front of the cycloalkylthio, for example, C3-C8 cycloalkylthio means that the cycloalkyl in the cycloalkylthio has 3-8 carbon atoms. Representative examples of cycloalkylthio comprise but are not limited to cyclopropylthio, cyclobutythio, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples of haloalkoxyl comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above, for example, C1-C6 haloalkylthio refers to a haloalkylthio having 1-4 carbon atoms. Representative examples of haloalkylthio comprise but are not limited to monofluoromethylthio, monofluoroethylthio, difluorobutylthio, or the like.

The term "heterocycloalkane ring" refers to fully saturated or partially unsaturated ring (comprising but not limited to such as 3-7 membered monocyclic ring, 7-11 membered bicyclic ring, or 8-16 membered tricyclic ring) , at least one heteroatom is present in a ring with at least one carbon atom. When the number of members is limited in front of the heterocycloalkane ring, it refers to the number of ring atoms in the heterocycloalkane ring, for example, 3-16 membered heterocycloalkane ring refers to a heterocycloalkane ring having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Heterocycloalkane ring can be attached to any heteroatom or carbon atom residue of ring or ring system molecule. Representative examples of monocyclic heterocycloalkyl comprise but are not limited to azetidine ring, oxetane ring, imidazoline ring, tetrahydrofuran ring, piperidine ring, piperazine ring, 2-oxypiperazine ring, 2-oxypiperidine ring, 4-piperidone ring, tetrahydropyran ring, morpholine ring, thiomorpholine ring, thiomorpholine sulfoxide ring, thiomorpholine sulfone ring, 1,3-dioxane ring, and tetrahydro-1,1-dioxothiophene ring. Polycyclic heterocycloalkane ring comprises spiro, fused and bridged heterocycloalkane ring, the spiro, fused and bridged heterocycloalkyl is optionally linked with other rings by single bond, or further linked with other cycloalkyl rings and heterocyclic rings by any two or more atoms on the ring.

The term "heterocycloalkyl" refers to fully saturated or partially unsaturated cyclic group (comprising but not limited to such as 3-7 membered monocyclic ring, 7-11 membered bicyclic ring, or 8-16 membered tricyclic ring), at least one heteroatom is present in a ring with at least one carbon atom. When the number of members is limited in front of the heterocycloalkyl, it refers to the number of ring atoms in the heterocycloalkyl, for example, 3-16 membered heterocycloalkyl refers to a heterocycloalkyl having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Heterocycloalkyl can be attached to any heteroatom or carbon atom residue of ring or ring system molecule. Representative examples of monocyclic heterocycloalkyl comprise but are not limited to azetidinyl, oxetanyl, imidazolidinyl, tetrahydrofuranyl, piperidinyl, piperazinyl, 2-oxypiperazinyl, 2-oxypiperidinyl, 4-piperidone group, tetrahydropyranyl, morpholine, thiomorpholine, thiomorpholine sulfoxide, thiomorpholine sulfone, 1,3-dioxyl, and tetrahydro-1,1-dioxothiophene. Polycyclic heterocycloalkyl comprises spiro, fused and bridged heterocyclyl, the spiro, fused and bridged heterocycloalkyl is optionally linked with other groups by single bond, or further linked with other cycloalkyl rings and heterocyclic rings by any two or more atoms on the ring.

The term "aromatic ring" refers to an all carbon monocyclic ring or fused polycyclic ring (i.e., a ring that share adjacent carbon atom pairs) with a conjugated π electron system, which is aromatic cyclic hydrocarbon compound. When the number of carbon atoms is limited in front of the aromatic ring, for example, C6-C12 aromatic ring means that the aromatic ring has 6-12 ring carbon atoms, such as benzene ring and naphthalene ring. The aromatic ring can be fused with other cyclic groups (comprising saturated or unsaturated rings), but cannot contain heteroatoms such as nitrogen, oxygen, or sulfur, and position connecting the parent must be on the carbon atom of the ring with a conjugated π electron system. The representative examples of aromatic ring are benzene ring and naphthalene ring, or the like.

The term "aryl" refers to an all carbon monocyclic ring or fused polycyclic ring (i.e., a ring that share adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl. The aromatic ring can be fused with other carbon ring (comprising saturated or unsaturated rings), but cannot contain heteroatoms such as nitrogen, oxygen, or sulfur, and position connecting the parent must be on the carbon atom of the ring with a conjugated π electron system. The representative examples of aryl comprises but are not limited to:

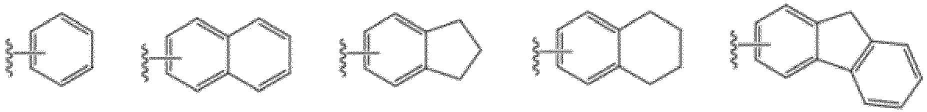

The term "heteroaromatic ring" refers to aromatic heterocyclic ring having one to more (preferably 1, 2, 3 or 4) heteroatoms, the heteroaromatic ring can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaromatic ring, it refers to the number of ring atoms of the heteroaromatic ring, for example, 5-12 membered heteroaromatic ring refers to a heteroaromatic ring having 5-12 ring atoms. Representative examples comprise but are not limited to pyrrole, pyrazole, imidazole, oxazole, isoxazole, thiazole, thiadiazole, isothiazole, furan, pyridine, pyrazine, pyrimidine, pyridazine, triazine, triazole, and tetrazolel, etc.

The term "heteroaryl" refers to aromatic heterocyclic ring group having one to more (preferably 1, 2, 3 or 4) heteroatoms, the heteroaryl can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms of the heteroaryl, for example, 5-12 membered heteroaryl refers to a heteroaryl having 5-12 ring atoms. Representative examples comprise but are not limited to pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazinyl, triazolyl and tetrazolyl, etc.

As used herein, the term "carboxyl" refers to -COOH or -alkyl-COOH, the alkyl is as defined above. For example, "C2-C4 carboxyl" refers to -C1-C3 alkyl-COOH. Representative examples of carboxyl comprise but are not limited to -COOH, - CH₂COOH, - C₂H₄COOH, or the like.

As used herein, the term "ester group" refers to R-C(O)-O- or -C(O)-O-R, wherein, R is alkyl, the alkyl is defined as above. For example, "C2-C4 ester group" refers to C1-C3 alkyl-C(O)-O- or -C (O)-O-C1-C3 alkyl. Representative examples of ester groups comprise but are not limited to CH₃COO-, C₂H₅COO-, C₃H₈COO-, (CH₃)₂CHCOO-, -COOCH₃, -COOC₂H₅, -COOC₃H₈,-COOC(CH₃)₃, or the like.

As used herein, the term "amide group" refers to R-C(O)-N- or -C(O)-N-R , wherein, R is alkyl, the alkyl is defined as above. For example, "C2-C4 amide group" refers to C1-C3 alkyl-C(O)-N- or -C(O)-N-C1-C3 alkyl. Representative examples of amide groups comprise but are not limited to CH₃CO-N-, C₂H₅CO-N-, C₃H₈CO-N-, (CH₃)₂CHCO-N-, -CO-N-CH₃-, -CO-N-CzHs, -CO-N-C₃H₈, or the like.

As used alone or as part of other substituent, the term "amino" refers to -NH₂.

As used alone or as part of other substituent, the term 'nitro' refers to -NO₂.

As used alone or as part of other substituent, the term "cyano" refers to -CN.

As used alone or as part of other substituent, the term "hydroxyl" refers to -OH.

As used alone or as part of other substituent, the term "sulfhydryl" refers to -SH.

In present invention, it should be understood that all substituents are unsubstituted, unless explicitly described herein as "substituted". The term "substituted" means that one or more hydrogen atoms on the specified group are substituted by specified substituent. The specific substituent is the substituent as described above, or the substituent in each example. Preferably, the "substituted" means that one or more (preferably 1. 2, 3, 4, 5, 6, 7, or 8) hydrogen atoms on the group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, 5-10 membered heteroaryl, 5-10 membered heterocycloalkyl. Unless otherwise specified, each substituted group can have a substituent selected from a specified group at any substituted position of the group, the substitution can be the same or different at each substituted position.

In the present invention, the term "prevention" refers to a method of preventing the occurrence of disease and/or its accompanying symptoms, or protecting a subject from getting disease. The term 'prevention' also comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100% inhibition, elimination and reversal. In some embodiments, compared to the level observed in the absence of the compound of present invention, the compound of present invention of the present invention alleviates, inhibits and/or reverses tumor and its accompanying symptoms such as by at least about 10%, at least about 30%, at least about 50%, at least about 80%, at least about 90% or 100%.

### Compound

As used herein, the terms "compound of the present invention", "isoquinoline compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof. It should be understood that the term also comprises a mixture of the above components.

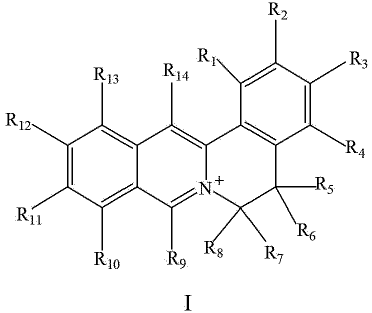

Specifically, the compound of formula I is as described above in the first aspect of the present invention.

The study of the present invention shows that the compound of the present invention has more significant inhibitory effect on tumor with low or no expression of NNMT gene, high expression of DNA methylase, high expression of UHRF1, high methylation level of nucleotide site of NNMT gene, and/or high methylation level of DNA CpG site of NNMT gene. The tumor with low or no expression of NNMT gene, high expression of DNA methylase, high expression of UHRF1, high methylation level of nucleotide site of NNMT gene, and/or high methylation level of DNA CpG site in promoter region of NNMT gene is sensitive to the compound of present invention.

The compound of formula I of the present invention is in the form of cation, and can form a pharmaceutically acceptable salt with an acid.

The term "pharmaceutically acceptable salt" refers to a salt formed by a compound of the present invention and an acid, the salt is suitable for use as a drug. Pharmaceutically acceptable salts comprises inorganic salts and organic salts. A preferred type of salt is the salt formed by the compound of the present invention and an acid. Acids suitable for salt formation comprise but are not limited to inorganic acid such as hydrochloric acid, hydrobromic acid, hydrofluoric acid, sulfuric acid, nitric acid, phosphoric acid and the like; organic acid such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid, and acidic amino acid such as aspartic acid and glutamic acid.

The compound of formula I in present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

In another preferred embodiment, the compound of formula I has the following structure of formula 1-1: 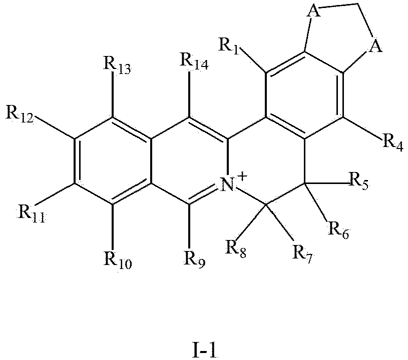 wherein,
A is O or S;
R₁, R₄, R₅, R₆, R₇, R₈, R₉, R₁₀, R₁₁, R₁₂, R₁₃ and R₁₄ are as described above in the first aspect of the present invention;
R₁₀ is hydrogen, R17-A -,
A is O or S;
R₁₇ is substituted or unsubstituted C3-6 alkyl, C2-C4 alkenyl-C1-C2 alkyl-, C2-C8 ester group-C1-C2 alkyl-.

The preferred compounds of the present invention are selected from the following group: 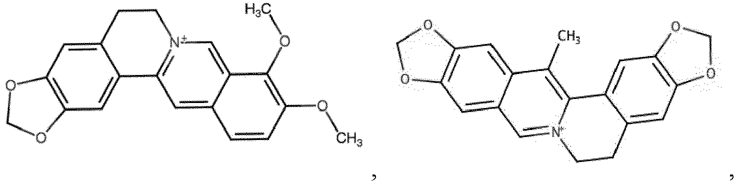 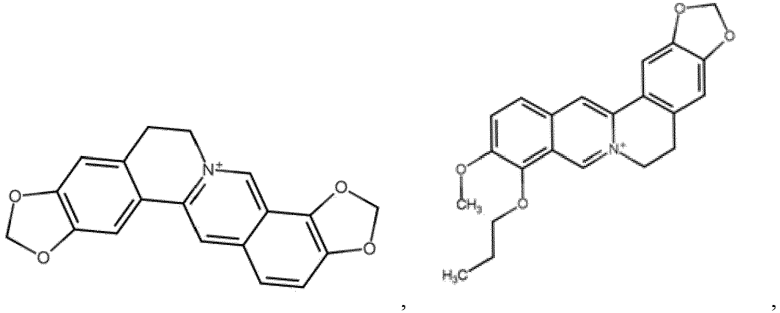 coptisine

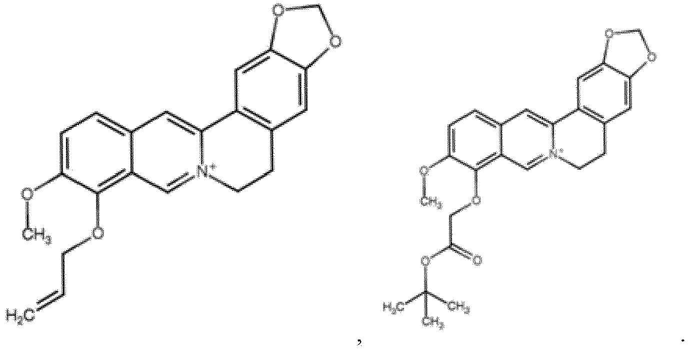

The preferred compounds of the present invention are selected from the following group: 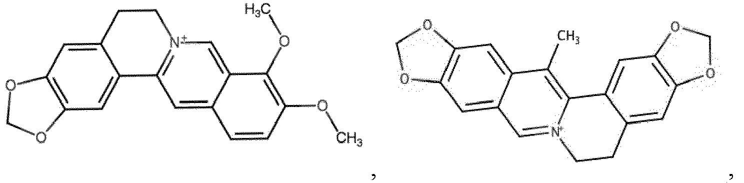 worenine 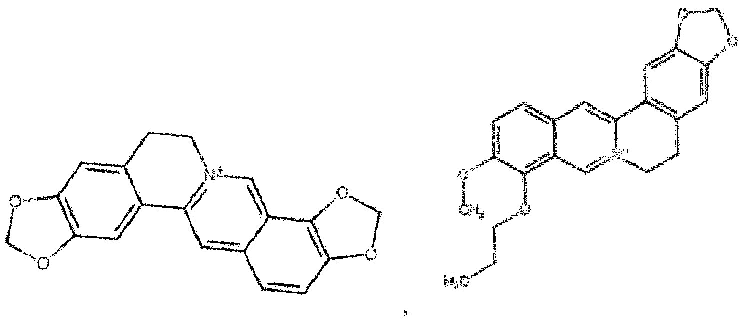 coptisine

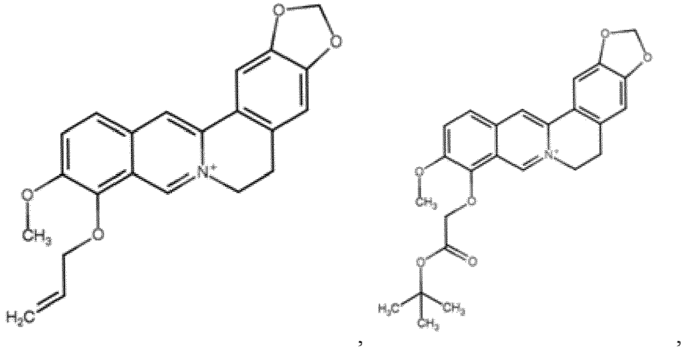

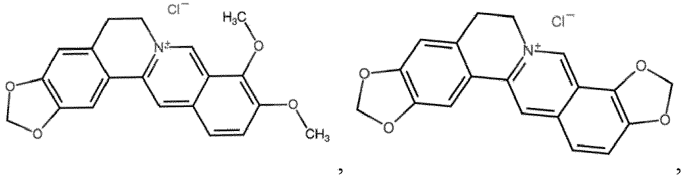

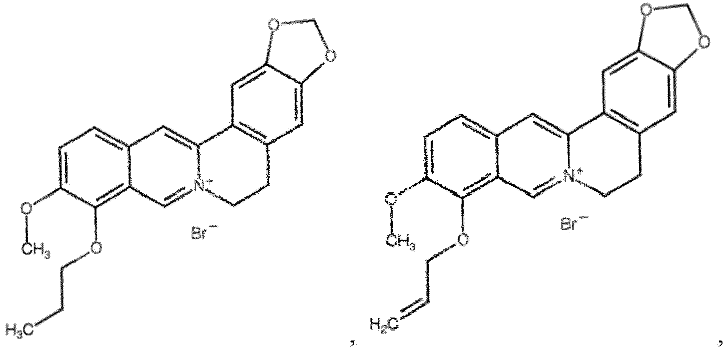

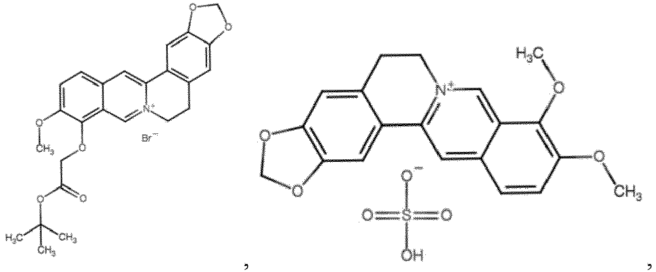

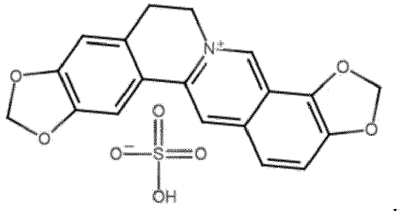

In another preferred embodiment, the berberine comprises berberine hydrochloride or berberine sulfate.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF 000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) to the transcription start site itself and 499 bp after the transcription start site (underlined section) in NNMT gene, the total length of promoter region of NNMT gene is 2500 bp, The nucleotide sequence of the promoter region of NNMT gene is as shown in SEQ ID NO: 1 as follows:
**SEQ ID NO: 1:** 
TATCCAAGAGCTATCAGCACTCCCATGTTTATTGTAGCACTGTTCACAATA
GCCAAGATTTGGAAGTACTCTAAGTGTCCATTAGCAGATGAATGGATAAAGAC
AATGTGGTAATACACATAATGGAGTACTATTCAGTCATAAAGAAGAATTAGAT
CCTGTCATTTGCAATAACATGGATGGAACTGGAGGTCATAATGTTGAGTGAAA
TAAACCAGGCACAGAAAGACAAACTTTGCATGTTCTCACTTATTTATGGGAGC
TAAAAACTAAAATAACTGAACTCACAGAGATAGAGAGTAGAAGGATGGTTACG
AGAGGATGGGAAGGGTAGCGAGGTGGGTAGGGGGGATGTGGGGATCATTAA
TGGGTATAAAAAATAGTTAGAGGCCAGGCGCAGTGGCTCACGCCTGTAATCC
CAGCACTTTGGGAGGCCGAGGTAGGCGGAACACCTGAGGAGTTCAAGACCAG
CCTGGCCAATATGATGAAACCCCGTCTCTACTAAAAATACAAAAATTAGCTGG
GCGTGATGGTGTGCACCTGTAGTCCCAGCTGCTTGGGAGGCTGAGGCAGGAG
AATCGCTGGAACCCAAGAGGTGAAGGTTGCAGTGAGCTGAGATCGCGTCACT
GCACTCCAGCCTGGGTGACAGAGTGAGACTCCACATCAAAAAAAAAAAAAAA
AAGTTAGAAAGATTGAATAAGACCTAATATTTGCTAGCACAACAGGGTGAATA
TAGTAAAAAATAATTTATTTGTACCTTCAAAAATAACTAGACAAGTATAATTGG
GTTGTTTGTAACACACAAAAAATAAGTACTTGAAGTGGTGGATACCCCATTTA
CCCTGATGTGATTATTTTGTATTGCAGGCCTCTATCAGAATATCTCATGTAACC
CATAAATATATACACCTACTCTGTACCCACAAAAAGTTTTTAAAAAGAAAAATA
AATAGCAACCGAAAAAAAAAGAGAGGGAGAAAAGAAAAAAGAAAAAAAAATC
AAGTGCCTGGCTGGGTAGAATAAATTCTAAGGCCACAATGTTACTGACCATGG
GTTTTTTGGCTCTCAGTGTATAGAAATTGACACAAGGCCAATAGTCTTCCCAA
ACATGCTTTACTGGAACTTACGCCCTGGCATAAGGGCCACAACAAAAGAGAGA
GCGAATTCTCTGGCTTGCTGACTCCTTGGAAAAAACCGGTAGGGATTTTTTTA
TTAGGCAAAGCACAGGAATTGACGTCAGAGGCAGGATGTGCTGCTGGGCAAA
GCATACGAGAAGTGGGGTATGCAGGTCAGCATTACTTGGTTGCAATGGTTATC
TTGAGGAATGGGCCAACTGGTGGTCTGGCCAGTGGCAACAAGGCTGTAAATC
AATTATTCAGCATTCCTTCCCAAGGTGGGACACCCGGCAACATTGTTTATCTC
CTAAGGCCAGTTCCTGGAATTAAGTGAAAGGATGACTAATGGACATGTTGTCA
GTGAGGTAGTGGTGTGGGTTTTGTGACCAGTGGGAATGCACGAAAGAATGCT
TTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGA
CAGAGTCTTGCTCTGTCGCCCAGGCTGGAGTGCAGTGGCGCCATCTTGGCTC
ACTGCAAGCTCCGCCCCCCGGGTTCATGCCATTCTCCTGCCTCAGCCTCCCAA
GTAGCTGGGACTGCAGGTGCCTACCACCAAGCCCAGCTAATTTTTTGTATTTT
TTTTTTAGTAGAGACGGGGTTTCACCATGTTAGCCAGGATGGTCTCGATCTCC
TGACCTCGTGATCTGCCCGCCTTAGCCTCCCAAAGTGCTGGGATTACAGGCAT
GAGCCTCCGCCCCCGGCCTTAAATAATTCTTAAAGGAAGTAAAGTTAACTTTG
 
AAAGAACTATCAGGATTTGGATTGACTGAAAGGAGTGGGGAAGCTTAGGGAG
GAGGTGCTTGCCAGACACTGGGTCATGGCAGTGGTCGGTGAAGCTGCAGTTGCCTA
GGGCAGGGATGGAGAGAGAGTCTGGGCATGAGGAGAGGGTCTCGGGATGTTTGGC
TGGACTAGATTTTACAGAAAGCCTTATCCAGGCTTTTAAAATTACTCTTTCCAGACT
TCATCTGAGACTCCTTCTTCAGCCAACATTCCTTAGCCCTGAATACATTTCCTATCC
TCATCTTTCCCTTCTTTTTTTTCCTTTCTTTTACATGTTTAAATTTAAACCATTCTTCG
TGACCCCTTTTCTTGGGAGATTCATGGCAAGAACGAGAAGAATGATGGTGCTTGTT
AGGGGATGTCCTGTCTCTCTGAACTTTGGGGTCCTATGCATTAAATAATTTTCCTGA
CGAGCTCAAGTGCTCCCTCTGGTCTACAATCCCTGGCGGCTGGCCTTCATCCCTTGG
GCAAGCATTGCATACAGCTCATGGCCCTCCCTCTACCATACCC.
 the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

In the present invention, the nucleotide sites from 840 bp to 469 bp before the transcription start site in NNMT gene is sites 1161-1532 of nucleotide sequence as shown in SEQ ID NO: 1.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Corresponding to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plants and animals. DNA methylation, as a relatively stable modification state, can be passed on to new generations of DNA during DNA replication process under the action of DNA methyltransferase, which is an important epigenetic mechanism.

There are two types of DNA methylation reactions. One type is that the DNA with two unmethylated strands is methylated, which is called denovo methylation; The other type is that the unmethylated strand of double-stranded DNA with one methylated strand and one unmethylated strand is methylated, which is called maintenance methylation.

Typically, DNA methylation is the methylation of DNA CpG site. The distribution of CpG binucleotide is very uneven in the human genome, while CpG remains or is higher than normal level in some regions of the genome. The CpG site enrichment region (also known as CpG island) is mainly located in the promoter region and exon regions of the gene, which is a region rich in CpG dinucleotide. About 60% of the promoters of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of the gene. Compared to the regulation of gene expression by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is an ideal biomarker.

### Use

The present invention provides a use of the compound of the present invention in the prevention and/or treatment of tumor.

### Tumor

The studies of the present invention shows the compound of present invention can be used for preventing and treating tumor.

As used herein, the term "tumor" and "cancer" are used interchangeably.

In a preferred embodiment of the present invention, the tumor comprises tumor with low or no expression of NNMT gene. Typically, the tumor with low or no expression of NNMT gene is as described above in the first aspect of the present invention.

In a preferred embodiment of the present invention, the tumor comprises tumor with high expression of DNA methylase. Typically, the tumor with high expression of DNA methylase is as described above in the first aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the first aspect of the present invention.

In a preferred embodiment of the present invention, the tumor comprises tumor with high expression of DNMT3a. Typically, the tumor with high expression of DNMT3a is as described above in the first aspect of the present invention.

In a preferred embodiment of the present invention, the tumor comprises tumor with high expression of DNMT3b. Typically, the tumor with high expression of DNMT3b is as described above in the first aspect of the present invention.

In a preferred embodiment of the present invention, the tumor comprises tumor with high expression of UHRF 1 (ubiquitin-like with PHD and ring finger domain 1). Typically, the tumor with high expression of UHRF1 is as described above in the first aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the first aspect of the present invention.

In a preferred embodiment of the present invention, the tumor comprises tumor with high methylation level of DNA CpG site of NNMT gene. Typically, the tumor with high methylation level of DNA CpG site of NNMT gene is as described above in the first aspect of the present invention.

Specifically, the tumor of present invention is as described above in the first aspect of the present invention.

In present invention, the corresponding type of tumor cell lines are shown in Table 2:

**Table 2**

| Tumor cell line | The corresponding type of tumor |
|---|---|
| NCI-H82 | Human small cell lung cancer cell |
| G-401 | Human renal carcinoma Wilms cell |
| MDA-MB-453 | Breast cancer cell |
| SW48 | Human colon adenocarcinoma cell |
| GB-1 | Human glioblastoma cell |
| CFPAC-1 | Human pancreatic cancer cell |
| SF126 | Human glioblastoma multiforme cell |
| 786-O | Clear cell renal cell adenocarcinoma cell |
| D341 Med | Cerebellar medulloblastoma cell |
| Kelly | Brain neuroblastoma cell |
| NB-1 | Brain neuroblastoma cell |

### Marker

The present invention provides a marker for determining whether the compound of present invention is suitable for use in the prevention and/or treatment of patient tumor, the marker comprises the expression level of NNMT gene, the expression level of DNA methylase, the expression level of UHRF1, the methylation level of nucleotide site of NNMT gene, and/or the methylation level of DNA CpG site of NNMT gene.

In a preferred embodiment, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether the compound of present invention is suitable for use in the prevention and/or treatment of patient tumor, the method comprises as follows:
the compound of present invention is suitable for use in the prevention and/or treatment of patient tumor with low or no expression of NNMT gene, high expression of DNA methylase, high expression of UHRF1, high methylation level of nucleotide site of NNMT gene, and/or high methylation level of DNA CpG site of NNMT gene; and/or
the compound of present invention is not suitable for use in the prevention and/or treatment of patient tumor with high expression of NNMT gene, low expression of DNA methylase, low expression of UHRF1, low methylation level of nucleotide site of NNMT gene, and/or low methylation level of DNA CpG site of NNMT gene.

Specifically, the tumor with low or no expression of NNMT gene, high expression of DNA methylase (e.g, NNMT1), high expression of UHRF1, high methylation level of nucleotide site of NNMT gene, and/or high methylation level of DNA CpG site of NNMT gene is as described above in the first aspect of the present invention.

Specifically, the tumor with high expression of NNMT gene, low expression of DNA methylase (e.g, NNMT1), low expression of UHRF1, low methylation level of nucleotide site of NNMT gene, and/or low methylation level of DNA CpG site of NNMT gene is as described above in the second aspect of the present invention.

The present invention further provides a use of the biomarker or the expression level of the biomarker in the preparation of reagent kit for determining whether the compound of the present invention is suitable for use in the prevention and/or treatment of patient tumor.

### Composition or preparation, active ingredient combination, medical kit and administration method

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

Typically, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 1-10 µg/kg.bw. Of course, the specific dose should also take into account the route of administration, the patient's health and other factors, which are within the skill range of skilled doctors.

### The main advantages of the present invention comprise:

The invention provides a marker for guiding precise administration of the compound of present invention, the marker can be used to effectively identify tumor patients sensitive to such anti-tumor drugs, improve treatment effect of drug, and avoid administrating such drug to tumor patient insensitive to such anti-tumor drug, thus realizing the precise application of the compound of present invention.

The present invention has unexpectedly found the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of present invention is suitable for use in the treatment of specific tumor.

The tumor with low or no expression of NNMT gene, high expression of DNA methylase, high expression of UHRF1, high methylation level of nucleotide site of NNMT gene, and/or high methylation level of DNA CpG site of NNMT gene is highly sensitive to the compound of present invention, ie, the compound of present invention has significant inhibitory effect on tumor with low or no expression of NNMT gene, high expression of DNA methylase, high expression of UHRF1, high methylation level of nucleotide site of NNMT gene, and/or high methylation level of DNA CpG site of NNMT gene. Moreover, the detection method of methylation level of DNA CpG site is stable and reliable, which is suitable for the development of molecular markers.

The present invention will be further illustrated below with reference to the specific examples.

It should be understood that these examples are only to illustrate the invention but are not to limit the scope of the invention. The experimental methods with no specific conditions described in the following examples are generally performed under the conventional conditions, or according to the manufacturer's instructions. Unless indicated otherwise, parts and percentage are calculated by weight.

### Example

The structure of different isoquinoline compounds (berberine, berberine hydrochloride, worenine, coptisine hydrochloride, compound BBR-A1, compound BBR-A2, compound BBR-A5) were as follows:

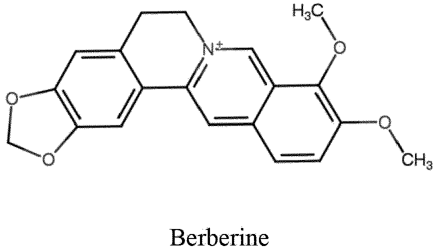

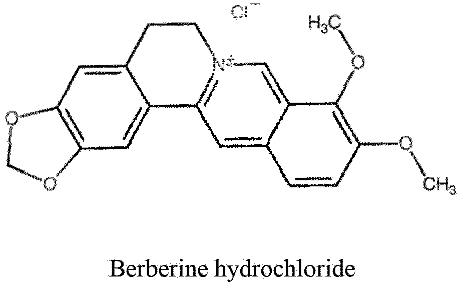

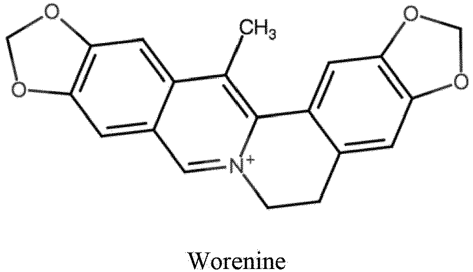

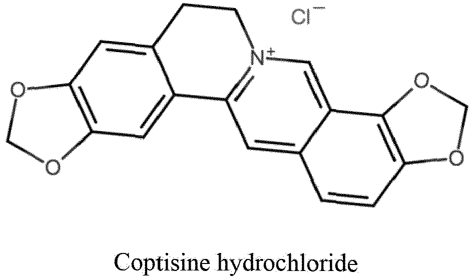

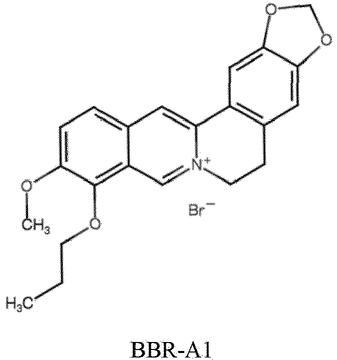

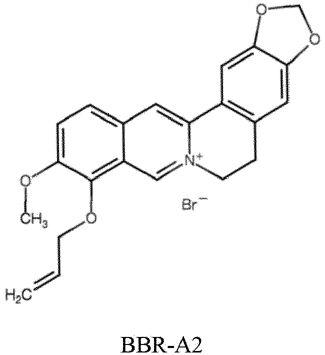

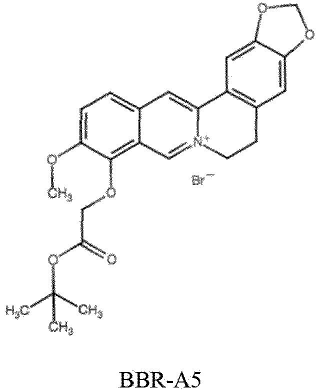

DNMT3a refers to DNA methyltransferase 3a, NCBI entrez gene: 1788; Uniprotkb/Swiss-port: Q9Y6K1.

DNMT3b refers to DNA methyltransferase 3b, NCBI entrez gene: 1789; Uniprotkb/Swiss-port: Q9UBC3.

DNMT1 refers to DNA methyltransferase 1, NCBI entrez gene: 1786; Uniprotkb/Swiss-port: P26358.

UHRF1 refers to ubiquitin-like with PHD and ring finger domain 1, NCBI entrez gene: 29128; Uniprotkb/Swiss-port:Q96T88.

NNMT refers to Nicotinamide N-Methyltransferase.

The nucleotide sequence of the promoter region of NNMT gene was as shown in SEQ ID NO: 1.

The nucleotide sites from 1050 bp before the transcription start site to 499 bp after the transcription start site in NNMT gene was sites 951-2500 of nucleotide sequence as shown in SEQ ID NO: 1.

The nucleotide sites from 1050 bp to 193 bp before the transcription start site in NNMT gene was sites 951-1808 of nucleotide sequence as shown in SEQ ID NO: 1.

The nucleotide sites from 840 bp to 469 bp before the transcription start site in NNMT gene was sites 1161-1532 of nucleotide sequence as shown in SEQ ID NO: 1.

### Example 1

### Preparation of Compound 1

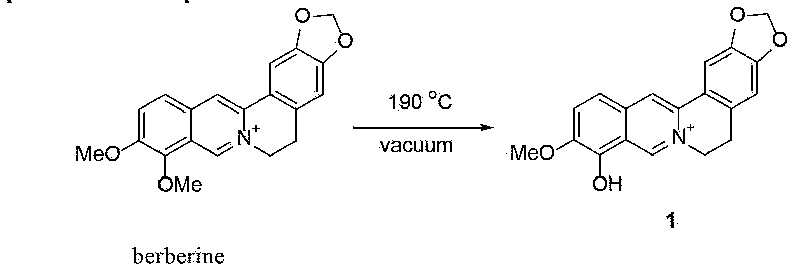

Berberine (12.0 g, 35.6 mmol) was added to a 100 mL single mouthed bottle, then vacuumed with oil pump. The mixture was slowly heated to 190-210 °C, and reacted for 2 h, the color of solid changed from yellow to deep red. The reaction was monitored until the raw material berberine was fully reacted using TLC (DCM: MeOH=10:1). The mixture was cooled to room temperature, the crude product was purified by silica gel column chromatography (eluent: DCM: MeOH=20:1) to afford compound 1 (8.2 g, yield 79%) as a dark red powder.
¹H NMR (400 MHz, CD₃OD) : δ 9.26 (s, 1H), 8.02 (s, 1H), 7.52 (d, J = 8.0 Hz, 1H), 7.43 (s, 1H), 6.88 (d, J = 8.0 Hz, 1H), 6.83 (s, 1H), 6.03 (s, 2H), 4.60 (t, J = 6.0 Hz, 2H), 3.87 (s, 3H),3.35 (t, J = 6.0 Hz, 2H), 3.14-3.11 (m, 2H).

### Example 2

### Preparation of Compound BBR-A1

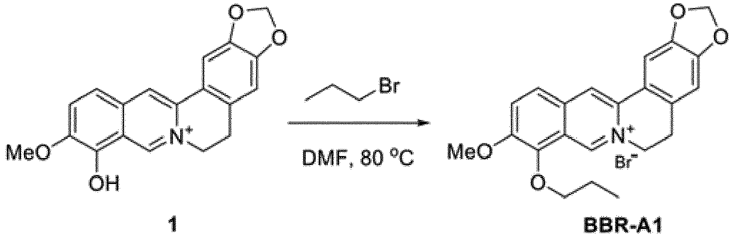

Compound 1 (200 mg, 0.62 mmol) and 1-bromopropane (305 mg, 2.48 mmol, 4.0 eq) were dissolved in DMF (3 mL), the mixture was stirred for 12h at 80 °C. The reaction was monitored until the raw material compound 1 was fully reacted using TLC (DCM: MeOH=10:1), then the solvent was removed by vacuum distillation, the crude product was purified by silica gel column chromatography (eluent: DCM: MeOH=20:1) to afford compound BBR-A1 (124 mg, 55%) as a yellow solid.
¹H NMR (400 MHz, CD3OD): δ 9.68 (s, 1H), 8.70 (s, 1H), 8.10 (d, J = 8.0 Hz, 1H), 8.00 (d, J = 8.0 Hz, 1H), 7.66 (s, 1H), 6.96 (s, 1H), 6.11 (s, 2H), 4.94 (t, J = 6.0 Hz, 2H), 4.37 (t, J = 6.0 Hz, 2H), 4.10 (s, 3H), 3.25 (t, J = 8.0 Hz, 2H), 1.97-1.93 (m, 2H), 1.13 (t, J = 7.2 Hz, 3H).
ESI-MS (m/z): calculated: C₂₂H₂₂NO₄ [M]⁺ 364.4, found: 364.0.

### Example 3

### Preparation of Compound BBR-A2

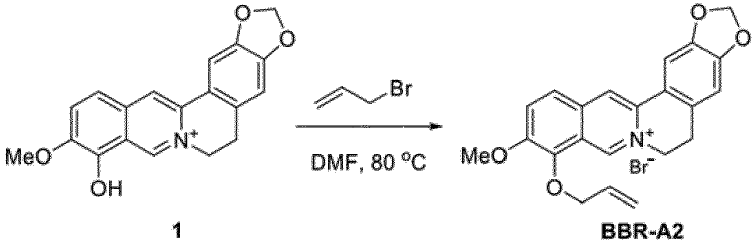

Compound 1 (200 mg, 0.62 mmol) and 1-bromopropylene (300 mg, 2.48 mmol, 4.0 eq) were dissolved in DMF (3 mL), the mixture was stirred for 12h at 80 °C. The reaction was monitored until the raw material compound 1 was fully reacted using TLC (DCM: MeOH=10:1), then the solvent was removed by vacuum distillation, the crude product was purified by silica gel column chromatography (eluent: DCM: MeOH=20:1) to afford compound BBR-A2 (121 mg, 54%) as a yellow solid.
¹H NMR (400 MHz, CD3OD) : δ 9.72 (s, 1H), 8.71 (s, 1H), 8.11 (d, J = 8.0 Hz, 1H), 8.01 (d, J = 8.0 Hz, 1H), 7.67 (s, 1H), 6.97 (s, 1H), 6.26-6.19 (m, 1H), 6.11 (s, 2H), 5.44 (d, J = 16.0 Hz, 1H), 5.28 (d, J = 16.0 Hz, 1H), 4.96-4.84 (m, 4H), 4.11 (s, 3H), 3.28-3.24 (m, 2H).
ESI-MS (m/z): calculated: C₂₂H₂₀NO₄, [M]⁺ 362.4, found: 362.0.

### Example 4

### Preparation of Compound BBR-A5

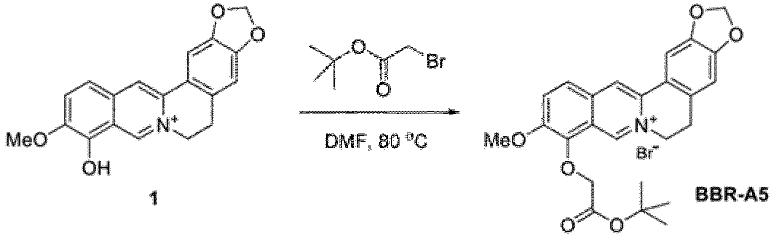

Compound 1 (200 mg, 0.62 mmol) and tert-butyl bromoacetate (484 mg, 2.48 mmol, 4.0 eq) were dissolved in DMF (3 mL), the mixture was stirred for 12h at 80 °C. The reaction was monitored until the raw material compound 1 was fully reacted using TLC (DCM: MeOH=10:1), then the solvent was removed by vacuum distillation, the crude product was purified by silica gel column chromatography (eluent: DCM: MeOH=20:1) to afford compound BBR-A5 (175 mg, 65%) as a yellow solid.
¹H NMR (400 MHz, CD3OD) : δ 9.97 (s, 1H), 8.71 (s, 1H), 8.12 (d, J = 8.0 Hz, 1H), 8.00 (d, J = 8.0 Hz, 1H), 7.67 (s, 1H), 6.99 (s, 1H), 6.11 (s, 2H), 5.03 (s, 2H), 4.96-4.93 (m, 2H), 4.10 (s, 3H), 3.29-3.26 (m, 2H), 1.46 (s, 9H).
ESI-MS (m/z): calculated: C₂₅H₂₆NO₆, [M]⁺ 436.5, found: 436.0.

### Example 5

The inhibitory effect of isoquinoline compounds on various tumor cell lines was detected using cell activity detection reagent.

### Experimental background:

Cell viability was detected using the Promega CellTiter-Glo kit, the kit reflected cell viability by directly measuring intracellular ATP content. In this experiment, the IC₅₀ values of isoquinoline compounds on various tumor cell lines was determined.

### Experimental method and result:

Tumor cells were cultured in 10% FBS- containing medium (+ p/s), and incubated for 3h or the following day, then the gradient diluted isoquinoline compound was added. After 3 days of culture, the relevant IC₅₀ (50% inhibiting concentration) was measured. The name, source and culture conditions of each tumor cell line were as follows:
Cell line NCI-H82 (ATCC, No. HTB-175) was cultured in 10% fetal bovine serum-containing RPMI1640 medium (+P/S).
Cell line G-401 (ATCC, No. CRL-1441) was cultured in 10% fetal bovine serum-containing McCoy's 5a medium (+P/S).
Cell line MDA-MB-453 (ATCC, No. HTB-131) was cultured in 10% fetal bovine serum-containing Leibovitz's L-15 medium (+P/S).
Cell line SW48 (ATCC, No. CCL-231) was cultured in 10% fetal bovine serum-containing Leibovitz's L-15 medium (+P/S).
Cell line CFPAC-1 (ATCC, No. CRL-1918) was cultured in 10% fetal bovine serum-containing IMDM medium (+P/S).
Cell line 786-O (ATCC, No. CRL-1932) was cultured in 10% fetal bovine serum-containing RPMI1640 medium (+P/S).
Cell line GB-1 (JCRB, No. IFO50489) was cultured in 10% fetal bovine serum-containing DMEM medium (+P/S).
Cell line SF126 (JCRB, No. IFO50286) was cultured in 10% fetal bovine serum-containing EMEM medium (+P/S).

The experiment result was shown in Table 3 below:

**Table 3 Inhibitory effect of different isoquinoline compounds on different cell lines (IC₅₀ µM)**

| Sensitivity | Cell line | Berberine hydrochloride | worenine | Coptisine hydrochloride | Compound BBR-A1 | Compound BBR-A2 | Compound BBR-A5 |
|---|---|---|---|---|---|---|---|
| Sensitive group | NCI-H82 | 0.51 | 4.3 | 1.8 | 0.4 | 0.7 | 0.5 |
| | G-401 | 1.12 | 2.3 | 3.4 | 1.0 | 1.6 | 1.1 |
| | MDA-MB-453 | 0.94 | 1.3 | 1.6 | 0.8 | 1.3 | 0.7 |
| | SW48 | 1.26 | 9.6 | 6.0 | 0.2 | 3.2 | 1.8 |
| | CFPAC-1 | 9.5 | >30 | 22.1 | 8.3 | 14.9 | 17.7 |
| | 786-O | 15.3 | >30 | >30 | 16.3 | 37.1 | 18.5 |
| | GB-1 | 19.6 | >30 | 24.8 | 9.1 | 11.2 | 9.2 |
| Insensitive group | SF-126 | 21.3 | >30 | >30 | 7.5 | 22.6 | 15.5 |

| | | | | | | | |
|---|---|---|---|---|---|---|---|
| Note: IC₅₀ refered to 50% inhibiting concentration, ie, the concentration of the inhibitor when 50% inhibitory effect was achieved. | | | | | | | |

The Table 3 showed the sensitivity of different cells to the isoquinoline compounds, NCI-H82 (human small cell lung cancer cell), G-401 (human renal carcinoma Wilms cell), MDA-MB-453 (breast cancer cell) and SW48 (human colon adenocarcinoma cell) were sensitive to isoquinoline compounds with low IC₅₀ value, while 786-O (clear cell renal cell adenocarcinoma cell), CFPAC-1 (human pancreatic cancer cell), GB-1 (human glioblastoma cell) and SF126 (human glioblastoma multiforme cell) were not sensitive to isoquinoline compounds with high IC₅₀ value.

### Example 6

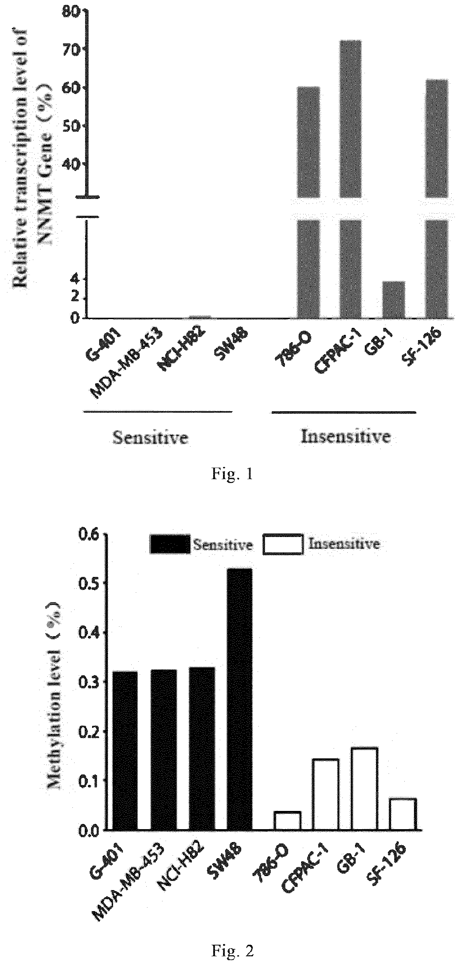

The mRNA transcription level of NNMT gene in four tumor cell lines sensitive to isoquinoline compounds and four tumor cell lines insensitive to isoquinoline compounds was measured using RT-qPCR gene expression analysis test, and the expression of NNMT gene in the tumor cell lines was measured respectively. The results were shown in Fig. 1.

As shown in Fig. 1, the mRNA transcription level of NNMT gene in four tumor cell lines (NCI-H82, G-401, MDA-MB-453, SW48) sensitive to isoquinoline compounds and four tumor cell lines (786-O, CFPAC-1, GB-1, and SF126) insensitive to isoquinoline compounds was measured using RT-qPCR gene expression analysis test, the results showed the expression of NNMT gene was low in sensitive cell lines (NCI-H82, G-401, MDA-MB-453 and SW48) , and the expression of NNMT gene was high in insensitive cell lines (786-O, CFPAC-1, GB-1 and SF126).

Therefore, the Fig. 1 showed that compared with tumor cell lines with high expression of NNMT gene, the inhibitory effect of isoquinoline compounds on tumor cell lines with low expression of NNMT gene was significantly enhanced, i.e, the expression of NNMT gene in tumor cell was negatively correlated with the sensitivity of tumor cell to isoquinoline compounds.

### Example 7

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in four tumor cell lines(NCI-H82, G-401, MDA-MB-453 and SW48) sensitive to isoquinoline compounds and four tumor cell lines (786-O, CFPAC-1, GB-1 and SF126) insensitive to isoquinoline compounds. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.2, Fig.3, and Fig.4.

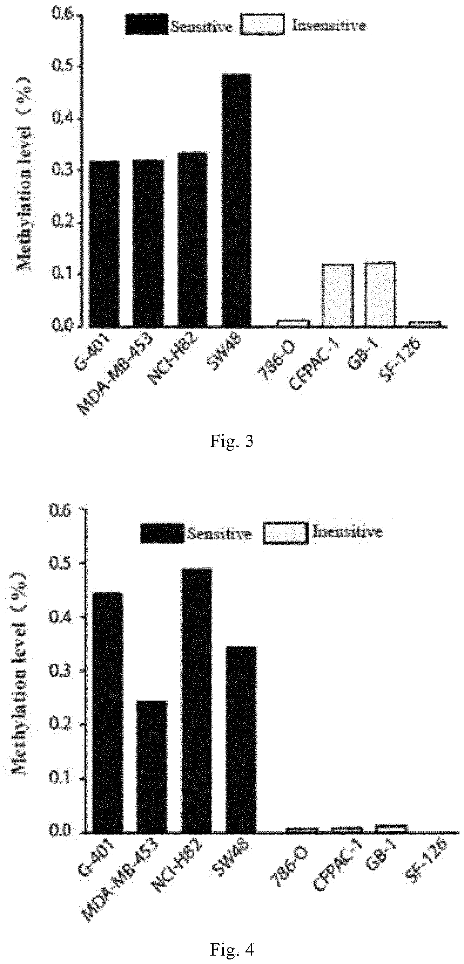

As shown in Fig.2 (the promoter region of NNMT gene), Fig.3 (the region from 1050 bp before the transcription start site to 499 bp after the transcription start site in NNMT gene) and Fig.4 (the region from 1050 bp to 193 bp before the transcription start site in NNMT gene), the isoquinoline compounds had significantly stronger inhibitory effect on tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, while the isoquinoline compounds had significantly weaker inhibitory effect on tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499bp after the transcription start site in NNMT gene and the region from 1050 bp to 193 bp before the transcription start site in NNMT gene was positive correlation with the sensitivity of tumor cell to to isoquinoline compounds.

### Example 8

The methylation level of specific DNA CpG sites from 840 bp (i.e., position 114165695 on human chromosome 11) to 469 bp (i.e., position 114166066 on human chromosome 11) before the transcription start site in NNMT gene in three tumor cell lines (NCI-H82, G-401 and SW48) sensitive to isoquinoline compounds and three tumor cell lines (786-O, CFPAC-1 and SF126) insensitive to isoquinoline compounds was studied.

Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, then PCR amplification and sequencing analysis were performed on the region using corresponding primers to measure the methylation level of CpG site in the DNA region.

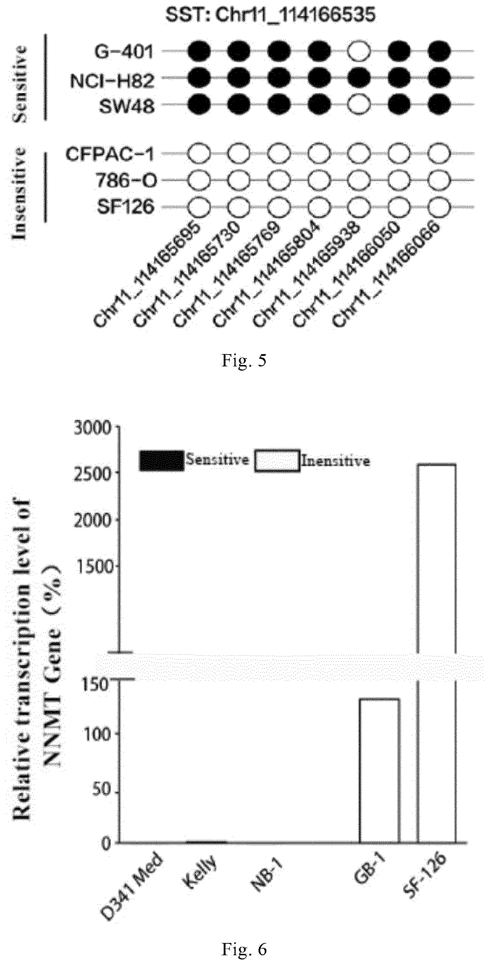

The study showed that almost all of the seven CpG sites (site 114165695, site 114165730, site 114165769, site 114165804, site 114165938, site 114166050 and site 114166066 on the human chromosome 11) in cell lines (NCI-H82, G-401 and SW48) sensitive to isoquinoline compounds were methylated, while none of the above seven CpG sites in cell lines (786-O, CFPAC-1 and SF126) insensitive to isoquinoline compounds were methylated, the methylation of related sites was shown in Fig.5.

The sites of the nucleotide sequence in SEQ ID NO: 1 corresponding to the site 114165695, site 114165730, site 114165769, site 114165804, site 114165938, site 114166050 and site 114166066 on the human chromosome 11 were as follows:

| The site on the human chromosome 11 | Corresponding to the sites of the nucleotide sequence in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### Example 9

The inhibitory effect of isoquinoline compounds on various brain tumor cell lines was detected using cell activity detection reagents.

### Experimental background:

Cell viability was detected using the Promega CellTiter-Glo kit, the kit reflected cell viability by directly measuring intracellular ATP content. In this experiment, the IC₅₀ values of isoquinoline compounds on various brain tumor cell lines was determined.

### Experimental method and result:

Brain tumor cells were cultured in 10% FBS- containing medium (+ p/s), and incubated for 3h or the following day, the gradient diluted isoquinoline compound was added. After 3 days of culture, the relevant IC₅₀ (50% inhibiting concentration) was measured. The name, source and culture conditions of each brain tumor cell line were as follows:
Cell line D341 Med (ATCC, No. HTB-187) was cultured in 20% fetal bovine serum-containing EMEM medium (+P/S).
Cell line Kelly (ECACC, No. 92110411) was cultured in RPMI1640 medium containing 10% fetal bovine serum and 2mM glutamine;
Cell line NB-1 (ATCC, No. HTB-131) was cultured in 10% fetal bovine serum-containing Leibovitz's L-15 medium (+P/S).

The experiment result was shown in Table 4 below:

**Table 4 Inhibitory effect of different isoquinoline compounds on different brain tumor cell lines (IC₅₀, µM)**

| Sensitivity | Cell line | Berberine hydrochloride | worenine | Coptisine hydrochloride | Compound BBR-A1 | Compound BBR-A2 | Compound BBR-A5 |
|---|---|---|---|---|---|---|---|
| Sensitive group | D341 Med | 4.5 | 4.8 | 2.8 | 1.0 | 1.7 | 1.5 |
| | Kelly | 4.1 | 3.9 | 4.4 | 1.3 | 2.1 | 1.8 |
| | NB-1 | 1.7 | 2.6 | 2.5 | 0.3 | 0.6 | 0.3 |
| Insensitive group | GB-1 | 19.6 | >30 | 24.8 | 9.1 | 11.2 | 9.2 |
| | SF-126 | 21.3 | >30 | >30 | 7.5 | 22.6 | 15.5 |

| | | | | | | | |
|---|---|---|---|---|---|---|---|
| Note: IC₅₀ refered to 50% inhibiting concentration, ie, the concentration of the inhibitor when 50% inhibitory effect was achieved. | | | | | | | |

The Table 4 showed the sensitivity of different brain tumor cells to the isoquinoline compounds, D341 Med (cerebellar medulloblastoma cell), Kelly (brain neuroblastoma cell) and NB-1 (brain neuroblastoma cell) were sensitive to isoquinoline compounds with low IC₅₀ value, while GB-1 (human glioblastoma cell) and SF-126 (human glioblastoma multiforme cell) were not sensitive to isoquinoline compounds with high IC₅₀ value.

### Example 10

The mRNA transcription level of NNMT gene in three brain tumor cell lines (D341 Med, Kelly and NB-1) sensitive to isoquinoline compounds and two brain tumor cell lines (GB-1 and SF126) insensitive to isoquinoline compounds was measured using RT-qPCR gene expression analysis test, and the expression of NNMT gene in the tumor cell lines was measured respectively. The results were shown in Fig.6.

As shown in Fig. 6, the mRNA transcription level of NNMT gene in three brain tumor cell lines (D341 Med, Kelly and NB-1) sensitive to isoquinoline compounds and two brain tumor cell lines (GB-1 and SF126) insensitive to isoquinoline compounds was measured using RT-qPCR gene expression analysis test, the results showed the expression of NNMT gene was low in sensitive cell lines (D341 Med, Kelly and NB-1) , and the expression of NNMT gene was high in insensitive cell lines (GB-1 and SF126).

Therefore, the Fig. 6 showed that compared with brain tumor cell lines with high expression of NNMT gene, the inhibitory effect of isoquinoline compounds on brain tumor cell lines with low expression of NNMT gene was significantly enhanced, i.e, the expression of NNMT gene in brain tumor cells was negatively correlated with the sensitivity of brain tumor cells to isoquinoline compounds.

### Example 11

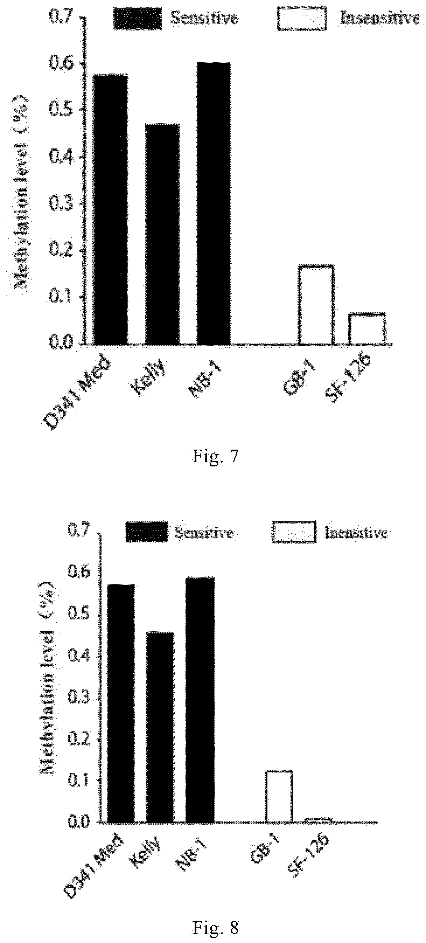

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in in three brain tumor cell lines (D341 Med, Kelly and NB-1) sensitive to isoquinoline compounds and two brain tumor cell lines (D341 Med, Kelly and NB-1) insensitive to isoquinoline compounds. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.7, Fig.8, and Fig.9.

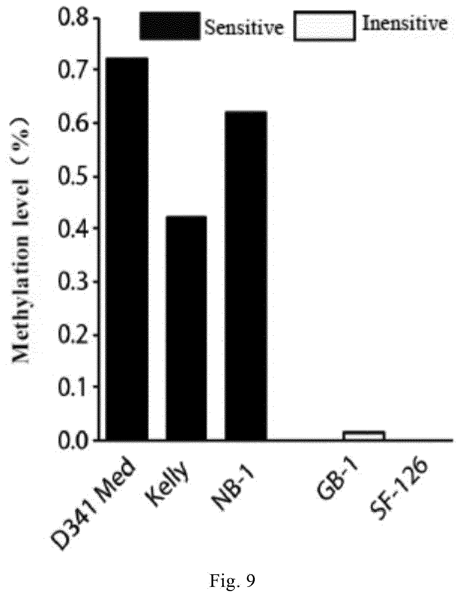

As shown in Fig.7 (the promoter region of NNMT gene), Fig.8 (the region from 1050 bp before the transcription start site to 499 bp after the transcription start site in NNMT gene) and Fig.9 (the region from 1050 bp to 193 bp before the transcription start site in NNMT gene), the isoquinoline compounds had significantly stronger inhibitory effect on brain tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, while the isoquinoline compounds had significantly weaker inhibitory effect on brain tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499bp after the transcription start site in NNMT gene and the region from 1050 bp to 193 bp before the transcription start site in NNMT gene was positive correlation with the sensitivity of brain tumor cells to isoquinoline compounds.

### Example 12

The methylation level of DNA in cell was maintained by DNA methylation enzymes (DNMT3a, DNMT3b and DNMT1). The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumor was determined in the Example.

### Experiment method and result:

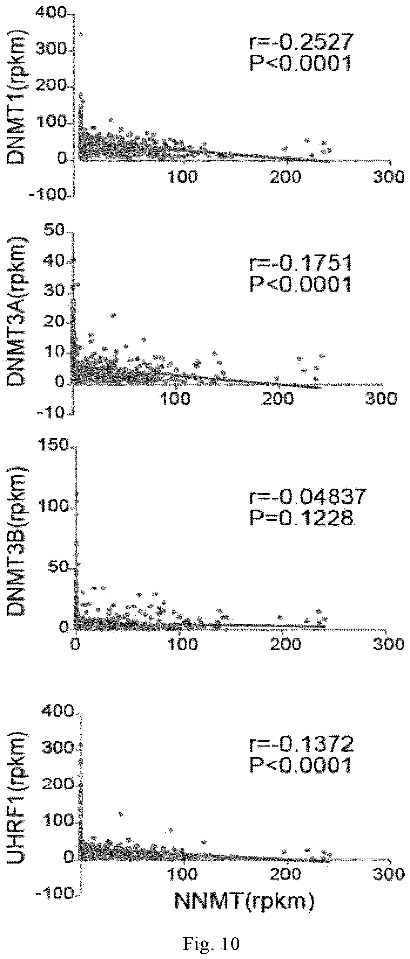

The expression of NNMT gene, DNMT1, UHRF1, DNMT3a, and DNMT3b in various cells were obtained from a public database (Cancer Cell Line Encyclopedia, CCLE, 1019 cells in total). Then, the correlation between expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.10.

The Fig.10 showed the expression of NNMT was negative correlation with the expression of DNA methylase and UHRF1 in each cell.

### Example 13

The expression level of NNMT in cells was significantly negative correlation with the sensitivity of the cell to isoquinoline compounds, and the expression level of DNMT1 gene was significantly positive correlation with the sensitivity of cell to isoquinoline compounds. The role of NNMT and DNMT1 in the sensitivity of cells to isoquinoline compounds was further determined.

### Experiment method and result:

The NCI-H82 cell overexpressing NNMT protein was obtained by inserting the NNMT gene into NCI-H82 cell using a viral vector. The expression of DNMT1 in NCI-H82 cells was knocked down using shRNA transfection. The changes in sensitivity of cell to isoquinoline compounds was investigated using intracellular ATP detection methods after overexpression of NNMT protein and/or knockdown of DNMT1 expression. The experiment result was shown in Fig.11, wherein, "Vector" referred to NCI-H82 cell transfected with empty virus as control group.

The Fig. 11 showed after the NNMT protein of NCI-H82 cells sensitive to isoquinoline compounds was overexpressed alone (ov-NNMT as shown in Figs. ) or the DNMT1 expression of NCI-H82 cells was knocked down using two different shRNA targeting the DNMT1 gene respectively (sh-DNMT1 # 1 referred to a shRNA targeting the DNMT1 gene, and the DNA sequence of sh-DNMT1 # 1 was GATCCGGCCCAATGAGACTGACATCAATT CAAGAGATTGATGTCAGTCTCATTGGGCTTTTTG (SEQ ID No: 2); the sh-DNMT1 # 2 was another shRNA targeting the DNMT1 gene, and the DNA sequence of sh-DNMT1# 2 was GATCCGGGATGAGTCCATCAAGGAAGATTCAAGAGATCTTCCTTGATGGACTCATCCT TTTTTG (SEQ ID No: 3), the sensitivity of NCI-H82 cell to berberine hydrochlorid decreased. After the NNMT protein was overexpressed and the expression of DNMT1 was knocked down in NCI-H82 cell simultaneously (ov-NNMT/sh-DNMT1#1 and ov-NNMT/sh-DNMT1#2 as shown in the Figs), the sensitivity of NCI-H82 tumor cells to berberine hydrochlorid decreased more significantly.

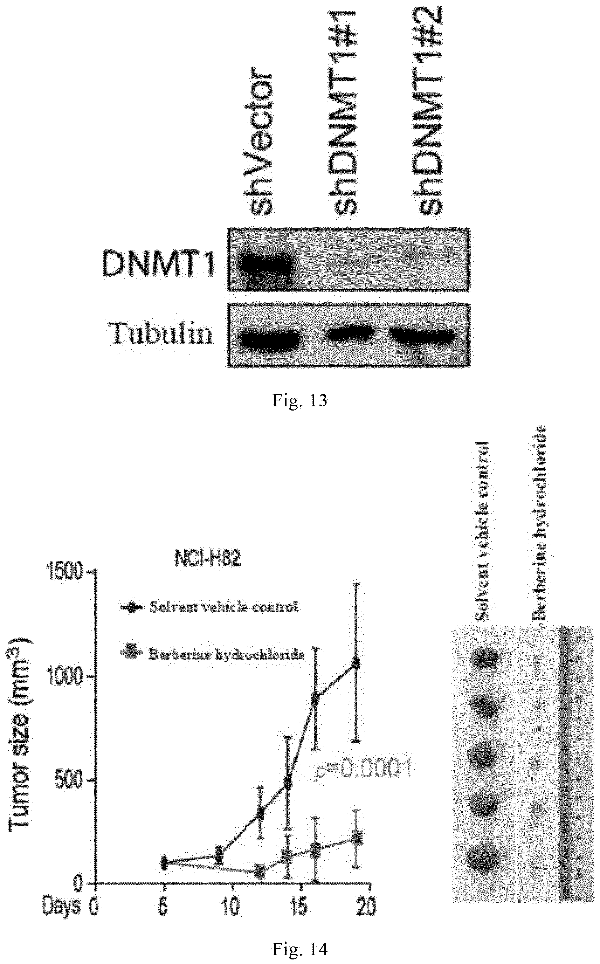

Compared to normal NCI-H82 (Vector), the NNMT protein content of NCI-H82 (ov-NNMT) overexpressing NNMT protein was detected using Western Blot assay, the result was shown in Fig. 12. Compared to normal NCI-H82 (shVector), the DNMT1 protein content of NCI-H82 with the knockdown of DNMT1 expression using two shRNA (sh-DNMT1 #1 or sh-DNMT1 #2) was detected by Western Blot assay, the result was shown in Fig. 13.

Therefore, the Example with the overexpression of NNMT protein and knockdown of DNMT1 expression in NCI-H82 cell further confirmed the expression level of NNMT in tumor cell was significantly negative correlation with the sensitivity of the tumor cell to isoquinoline compounds, while the expression level of DNMT1 in tumor cell was significantly positive correlation with the sensitivity of the tumor cell to isoquinoline compounds.

### Example 14

To verify whether the cells sensitive to isoquinoline compounds still maintained the sensitivity to isoquinoline compounds in vivo, inhibitory effect of berberine hydrochloride on tumor-bearing mice subcutaneously inoculated with sensitive cells (NCI-H82) was detected.

### Experiment method and result:

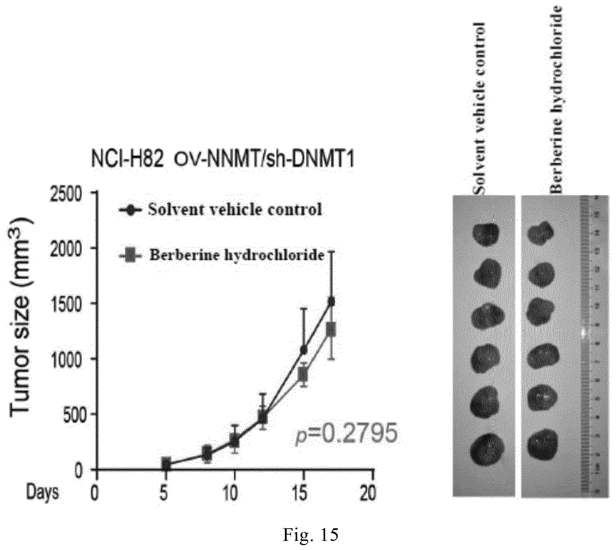

5×10⁶ of normal NCI-H82 cells, NCI-H82 cells with overexpression of NNMT protein and low overexpression of DNMT1 (i.e. ov-NNMT/sh-DNMT1, the method for overexpressing NNMT protein and knocking down DNMT1 expression was shown in Example 13) were subcutaneously inoculated in nude mice to establish tumor-bearing mice. Each group of tumor-bearing mice was injected intraperitoneally with the berberine hydrochloride at a dose of 10 mg/kg/day, the change of tumor volume size over time was measured, and the tumors were removed and photographed in each group of mice at the end of the experiment to investigate the inhibitory effect of berberine hydrochloride on the tumor. The mice in control group was injected intraperitoneally with solvent vehicle in the same method, and the tumor volume was calculated as follows: tumor volume=1/2 length × width ². The experiment result was shown in Fig. 14 and Fig. 15.

As shown in Fig.14 and Fig.15, the berberine hydrochloride could significantly inhibit the subcutaneous tumor of nude mice inoculated with NCI-H82, while the inhibitory effect of the berberine hydrochloride on the subcutaneous tumor of nude mice inoculated with ov-NNMT/sh-DNMT1 was significantly weaker than that of the berberine hydrochloride on the subcutaneous tumor of nude mice inoculated with NCI-H82, indicating that the berberine hydrochloride had a stronger inhibitory effect on tumor with low expression of NNMT, ie, tumor with low expression of NNMT was more sensitive to the isoquinoline compound (e.g., berberine hydrochloride), while tumor with high expression of NNMT and low expression of DNMT1 was less sensitive to the isoquinoline compound (e.g., berberine hydrochloride).

All documents mentioned in the present invention are incorporated herein by reference, as if each document is individually cited for reference. It should be understood that those skilled in the art will be able to make various changes or modifications to the present invention after reading the teachings of the present invention, which also fall within the scope of the claims appended hereto.

## Claims

1. A use of a compound of formula I, or an optical isomer thereof, or a racemate thereof, or a solvate thereof, or a pharmaceutically acceptable salt thereof in the preparation of a composition or a preparation for preventing and/or treating tumor 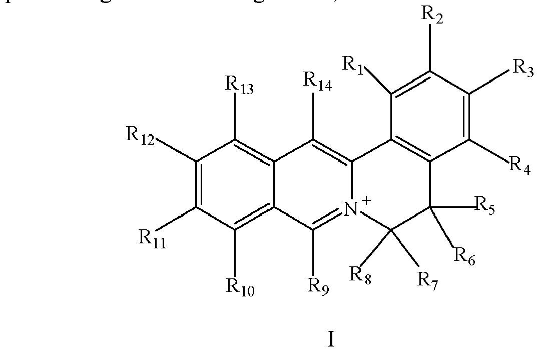 wherein,
R₁, R₂, R₃, R₄, R₁₀, R₁₁, R₁₂ and R₁₃ are each independently hydrogen, halogen, -CN, hydroxyl, sulfhydryl, nitro, amino, -COOH, -CHO, substituted or unsubstituted C1-C12 alkyl, substituted or unsubstituted C3-C12 cycloalkyl, substituted or unsubstituted C1-C12 alkoxyl, substituted or unsubstituted C1-C12 alkylthio, substituted or unsubstituted 3-12 membered heterocycloalkyl, substituted or unsubstituted C6-C12 aryl, substituted or unsubstituted 3-12 membered heteroaryl; or R₁ and R₂, R₂ and R₃, R₃ and R₄, R₁₀ and R₁₁, R₁₁ and R₁₂, and R₁₂ and R₁₃ each independently form substituted or unsubstituted C3-C12 cycloalkane ring, substituted or unsubstituted 3-12 membered heterocycloalkane ring, substituted or unsubstituted C6-C12 aromatic ring, or substituted or unsubstituted 3-12 membered heteroaromatic ring;
R₅, R₆, R₇, R₈, R₉ and R₁₄ are each independently hydrogen, halogen, - CN, hydroxyl, sulfhydryl, nitro, amino, -COOH, -CHO, substituted or unsubstituted C1-C12 alkyl, substituted or unsubstituted C3-C12 cycloalkyl, substituted or unsubstituted C1-C12 alkoxyl, substituted or unsubstituted C1-C12 alkylthio, substituted or unsubstituted 3-12 membered heterocycloalkyl, substituted or unsubstituted C6-C12 aryl, or substituted or unsubstituted 3-12 membered heteroaryl;
each "substituted" means that one or more (preferably 1, 2, 3, or 4) hydrogen atoms on the group are substituted by a substituent selected from the group consisting of C1-C8 alkyl, C3-C8 cycloalkyl, C2-C4 alkenyl, C2-C4 alkynyl, C1-C8 haloalkyl, C3-C8 halocycloalkyl, halogen, nitro, -CN, carbonyl (=O), cyano, hydroxyl, sulfhydryl, amino, C1-C4 carboxyl, C2-C8 ester group, C2-C4 amide group, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, 5-10 membered heterocycloalkyl, pyrrolidine-2,5-diketone group;
the heterocyclic ring of the heterocycloalkyl, heteroaryl, heterocycloalkane ring and heteroaromatic ring each independently contains 1-4 (preferably 1, 2, 3 or 4) heteroatoms selected from the group consisting of N, O and S.

2. The use of claim 1, wherein the compound of formula I is selected from the following group:

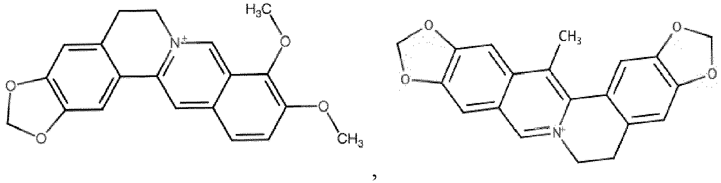

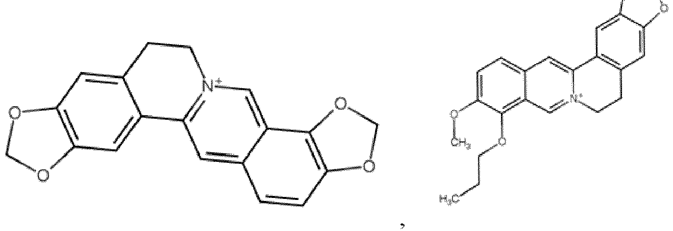

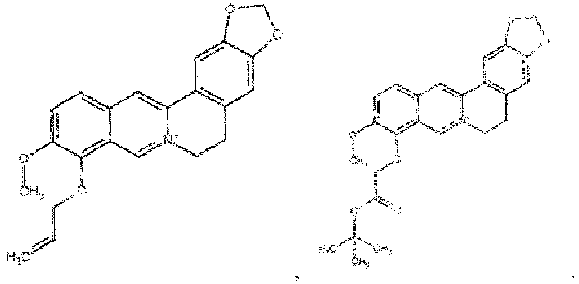

3. The use of claim 1, wherein the tumor comprises tumor with low or no expression of NNMT gene, and/or tumor with high methylation level of DNA CpG site of NNMT gene.

4. The use of claim 1, wherein the tumor comprises tumor with high expression of DNA methylase, tumor with high expression of UHRFl, and/or tumor with high methylation level of nucleotide site of NNMT gene.

5. The use of claim 3, wherein the methylation level of DNA CpG site of NNMT gene comprises the methylation level of DNA CpG site in promoter region of NNMT gene.

6. The use of claim 4, wherein the DNA methylase is selected from the group consisting of DNMT1, DNMT3a, DNMT3b, and combinations thereof.

7. The use of claim 1, wherein the tumor is selected from the group consisting of lung cancer, renal carcinoma, breast cancer, colon cancer, lymphoma, leukemia, pancreatic cancer, brain tumor, liver cancer, prostate cancer, and combinations thereof.

8. The use of claim 3, wherein the low or no expression of NNMT gene means the ratio (E1/E0) of the expression level E1 of NNMT gene in a cell ( e.g., tumor cell) to the expression level E0 of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

9. The use of claim 4, wherein the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

10. The use of claim 7, wherein the lung cancer is selected from the group consisting of non-small cell lung cancer, small cell lung cancer, and combinations thereof;
the colon cancer comprises colon adenocarcinoma;
the lymphoma is selected from the group consisting of B-cell lymphoma, skin T-cell lymphoma, and combinations thereof;
the brain tumor is selected from the group consisting of glioblastoma, neuroglioma, brain medulloblastoma, brain neuroblastoma, and combination thereof;
the renal carcinoma is selected from the group consisting of clear cell renal cell adenocarcinoma, renal carcinoma Wilms, and combination thereof; and/or
the leukemia is selected from the group consisting of T-lymphocyte leukemia, myeloid leukemia, and combinations thereof.

11. A marker for determining whether the compound of formula I, or an optical isomer thereof, or a racemate thereof, or a solvate thereof, or a pharmaceutically acceptable salt of claim 1 is suitable for use in the prevention and/or treatment of patient tumor, wherein the marker comprises the expression level of NNMT gene, the expression level of DNA methylase, the expression level of UHRF1, the methylation level of nucleotide site of NNMT gene, and/or the methylation level of DNA CpG site of NNMT gene.

12. A use of a detection kit in the preparation of concomitant diagnose kit for determining whether the compound of formula I, or an optical isomer thereof, or a racemate thereof, or a solvate thereof, or a pharmaceutically acceptable salt of claim 1 is suitable for use in the prevention and/or treatment of patient tumor;
the detection kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene.

13. A medicine kit, the medicine kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt of claim 1.

14. A method for preventing and/or treating tumor, which comprises administering the compound of formula I, or an optical isomer thereof, or a racemate thereof, or a solvate thereof, or a pharmaceutically acceptable salt of claim 1 to a subject in need.

15. A device or system, the device or system comprises:
(i) a detection module, the detection module is used to detect the expression level of NNMT gene, the expression level of DNA methylase, the expression level of UHRF1, the methylation level of nucleotide site of NNMT gene, and/or the methylation level of DNA CpG site of NNMT gene;
(ii) a output module, the output module comprises the output of the information as follows:
the compound of formula I, or an optical isomer thereof, or a racemate thereof, or a solvate thereof, or a pharmaceutically acceptable salt of claim 1 is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene; and/or
the compound of formula I, or an optical isomer thereof, or a racemate thereof, or a solvate thereof, or a pharmaceutically acceptable salt of claim 1 is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

16. A compound of formula I, or an optical isomer thereof, or a racemate thereof, or a solvate thereof, or a pharmaceutically acceptable salt thereof; 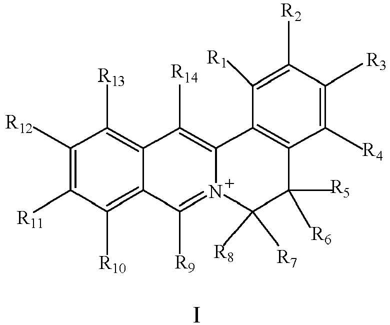 wherein,
R₁, R₂, R₃, R₄, R₅, R₆, R₇, R₈, R₉, R₁₀, R₁₁, R₁₂, R₁₃ and R₁₄ are as described in claim 1.

17. The compound of claim 16, the compound of formula I is selected from the following group:
